# EUROPÄISCHE PATENTSCHRIFT

(11) **EP 3 634 934 B1**
(45) Veröffentlichungstag und Bekanntmachung des Hinweises auf die Patenterteilung: **06.04.2022**
(21) Anmeldenummer: 18729353.5
(22) Anmeldetag: 24.05.2018
(51) Int. Cl.: C07C 209/16, C07C 211/10, C07C 213/02, C07C 215/08, B01J 23/00, B01J 8/00, B01J 19/00

(54) **VERFAHREN ZUR HERSTELLUNG VON ETHYLENAMINEN**
PROCESS FOR PREPARING ETHYLENAMINES
PROCÉDÉ POUR LA PRÉPARATION D'ÉTHYLÈNEAMINES

(30) Priorität: 09.06.2017 EP 17175151
(43) Veröffentlichungstag der Anmeldung: 15.04.2020
(73) Patentinhaber: BASF SE, 67056 Ludwigshafen am Rhein (DE)
(72) Erfinder: BEBENSEE, Regine Helga, 67056 Ludwigshafen (DE); HEIDEMANN, Thomas, 67056 Ludwigshafen (DE); BECKER, Barbara, 67056 Ludwigshafen (DE); KOCH, Eva, 67056 Ludwigshafen (DE); LUYKEN, Hermann, 67056 Ludwigshafen (DE); MELDER, Johann-Peter, 67056 Ludwigshafen (DE)
(74) Vertreter: BASF IP Association
(86) Internationale Anmeldenummer: PCT/EP2018/063589
(87) Internationale Veröffentlichungsnummer: WO 2018/224315

(56) Entgegenhaltungen:
- WO-A1-2005/110969
- WO-A1-2010/031719
- WO-A1-2013/072289
- CN-A- 102 233 272
- Na: "BASF: The right choice for nitric acid plants", BASF Technical Note, 1. Januar 2012 (2012-01-01), Seiten 1-12, XP055392817, Gefunden im Internet: URL:http://www.catalysts.basf.com/p02/USWe b-Internet/catalysts/en_GB/function/conver sions:/publish/content/microsites/catalyst s/prods-inds/process-catalysts/BF-9834_US_ N2O_and_DeNOX_Technote.pdf [gefunden am 2017-07-21]

## Beschreibung

Die vorliegende Erfindung betrifft ein Verfahren zur Herstellung von Alkanolaminen und Ethylenaminen, insbesondere Ethylendiamin.

Zur großtechnischen Herstellung von Ethylendiamin (EDA) kommen in der Regel zwei Verfahren zur Anwendung.

Zum einen kann EDA durch Umsetzung 1,2 Dichlorethan mit Ammoniak unter Abspaltung von HCl hergestellt werden (EDC-Verfahren). Ein weiteres großtechnisches Verfahren zur Herstellung von EDA ist die Umsetzung von Monoethanolamin (MEA) mit Ammoniak in Gegenwart von Aminierungskatalysatoren (MEA-Verfahren).

Alternativ zu den etablierten Verfahren kann die Herstellung von EDA auch durch Umsetzung von Monoethylenglykol (MEG) mit Ammoniak erfolgen.

Ein solches Verfahren hätte verschiedene Vorteile. Ein Vorteil besteht in der guten Verfügbarkeit von MEG im Vergleich zu MEA.

MEA wird großtechnisch durch Umsetzung von Ethylenoxid (EO) und Ammoniak hergestellt. Es entsteht in der Regel ein Reaktionsgemisch, welches neben MEA auch noch höhere Ethanolamine, wie Diethanolamin (DEOA) und Triethanolamin (TEOA) enthält. Diese Nebenprodukte müssen von MEA durch einen separaten Destillationsschritt abgetrennt werden. Ethylenoxid ist ein hochentzündliches Gas, welches mit Luft explosionsfähige Gemische bilden kann. Die Handhabung von EO ist entsprechend aufwendig. Die Herstellung von MEA erfordert somit eine technisch aufwendige EO-Anlage mit anschließender Reindestillation.

Demgegenüber kann MEG sowohl auf Basis von petrochemischen Rohstoffen als auch auf Basis von nachwachsenden Rohstoffen produziert werden. Petrochemisch wird MEG ebenfalls aus EO durch Umsetzung mit Wasser hergestellt. Ebenso wie bei der Umsetzung von EO mit Ammoniak, lässt sich bei der Umsetzung von EO mit Wasser nicht verhindern, dass bereits entstandenes MEG mit EO zu Nebenprodukten, wie Di- und Triethylenglykol, reagieren kann. Die Selektivitat von MEG liegt bei ca. 90% und ist somit aber deutlich höher als die Selektivität von MEA, welche in der Regel bei 70-80% liegt. Durch den Omega-Prozess der Schell konnte die Selektivität für MEG jedoch nochmals deutlich - auf ca. 99% -- gesteigert werden. Im Omega-Proess wird EO mit CO₂ zu Ethylencarbonat umgesetzt, welches im zweiten Schritt selektiv zu MEG hydrolysiert wird.

MEG lässt sich auch über den Synthesegasweg, z.B. durch oxidative Carbonylierung von Methanol zu Dimethyloxalat und dessen anschließender Hydrierung, herstellen. Damit kommt als weiterer petrochemischer Rohstoff auch Erdgas oder Kohle für die Herstellung von MEG in Frage.

Alternativ, kann MEG auch aus nachwachsenden Rohstoffen, wie Mais oder Zuckerrohr durch Fermentation zu Ethanol, anschließender Dehydratisierung zu Ethen und nachfolgender Umsetzung mit Sauerstoff zu Ethylenoxid hergestellt werden.

Aufgrund der vielen Herstellungsvarianten ist die Verfügbarkeit von MEG im Allgemeinen hoch, was sich in der Regel positiv auf die Rohstoffkosten auswirkt.

Im Stand der Technik wird offenbart, dass die Umsetzung von MEG mit Ammoniak zu EDA sowohl in der Flüssigphase als auch in der Gasphase erfolgen kann.

Die Aminierung von MEG in der Gasphase wird in den beiden chinesischen Anmeldungen CN 102 190 588 und CN 102 233 272 offenbart.

So beschreibt die CN 102 190 588 die einstufige Umsetzung von MEG und Ammoniak in Gegenwart von Cu-haltigen Katalysatoren. Der Reaktionsdruck liegt gemäß der Beschreibung in einem Bereich von 3 bis 30 bar. Die Reaktionstemperatur liegt im Bereich von 150 bis 350°C.

In der Anmeldung CN 102 233 272 wird die Umsetzung von MEG mit Ammoniak in der Gasphase an Katalysatoren offenbart, die Cu und Ni als Hauptbestandteile und Zr, Zn, Al, Ti, Mn und Ce als Nebenkomponente beinhalten. Die Zusammensetzung der erhaltenen Reaktionsgemische wurde allerdings nicht offenbart.

Alternativ zur Umsetzung in der Gasphase kann die Umsetzung von MEG mit Ammoniak und Wasserstoff auch in der Flüssigphase erfolgen. Das Reaktionsverhalten von Katalysatoren in der Gas- und Flüssigphase unterscheidet sich jedoch in der Regel erheblich, so dass Rückschlüsse von dem Reaktionsverhalten von MEG in der Gasphase auf das Reaktionsverhalten von MEG in der Flüssigphase im Allgemeinen nicht zulässig sind.

Eine Übersicht über die metallkatalysierte Aminierung von MEG in der flüssigen Phase wird in der Diplomarbeit "Reaktionskinetische Untersuchungen zur metallkatalysierten Aminierung von Ethylenglykol in der flüssigen Phase" von Carsten Wolfgang Ihmels gegeben ("Reaktionskinetische Untersuchungen zur metallkatalysierten Aminierung von Ethylenglykol in der flüssigen Phase", Diplomarbeit der Carl von Ossietzky Universität Oldenburg vom 17.03.2000). Ihmels beschreibt eine Vielzahl von Folge- und Nebenreaktionen, die bei der Aminierung von MEG auftreten können, beispielsweise die Bildung von Di- und Triethanolamin, Disproportionierung, Nitrilbildung, Carbonylkondensation und Fragmentierungsreaktionen. Kondensation und Dispoportionierung können bei zweiwertigen Alkoholen letztendlich auch zur Bildung von Oligomeren, wie Diethylentriamin (DETA), Triethylentetramin (TETA) und Polymeren führen. Eine weitere wichtige Nebenreaktion ist die Cyclisierung. So kann Diethanolamin oder DETA zum Piperazin (PIP) weiterreagieren. Höhere Temperaturen fördern eine sich an die Cyclisierung anschließende Dehydrierung zu Aromaten. Somit wird bei der Umsetzung von MEG mit Ammoniak ein breites Produktspektrum erhalten, wobei einige Produkte in dem Produktspektren kommerziell interessanter sind als andere. So ist für EDA, DETA und TETA der kommerzielle Bedarf höher als der von PIP oder Aminoethylethanolamin (AEEA). Gegenstand von vielen Untersuchungen bei der Umsetzung von MEG mit Ammoniak war es deshalb Katalysatoren und Reaktionsbedingungen zu finden, die zu einem vorteilhaften Produktspektrum führen.

Ihmels selber untersuchte die Umsetzung von MEG an Kobalt/Siliziumdioxid-Trägerkatalysatoren. Eine Aminierung zum gewünschten Zielprodukt MEA und EDA gelang nicht. Stattdessen bildeten sich hochpolymere Reaktionsprodukte. Unter milderen Bedingungen wurden bei noch unvollständigem Umsatz von MEG die Zielprodukte MEA und EDA in geringen Ausbeuten erhalten. Hauptprodukte waren oligomere Verbindungen.

US 4,111,840 offenbart die Umsetzung von MEG mit Ammoniak und Wasserstoff bei Drücken von 500 bis 5000 psig (ca. 34 bis 340 bar) an geträgerten Ni/Re-Katalysatoren. Vor der Reduktion werden die Katalysatoren im Bereich von 300 bis 500°C calciniert. Es wird offenbart, dass die Calcinierung unter einer Inertatmosphäre durchgeführt werden kann.

In der US 3,137,730 wird die Umsetzung von MEG mit Ammoniak in der Flüssigphase bei Temperaturen von 200-300°C und Drücken oberhalb von 1000 psig (ca. 69 bar) an Cu/Ni-Katalysatoren offenbart. In den Beispiele werden die Katalysatoren bei Temperaturen von 400 bis 800°C calciniert.

DE 1 172 268 offenbart die Umsetzung von Ethylenglykol an Katalysatoren, die mindestens eines der Metalle Cu, Ag, Mn, Fe, Ni und Co enthalten. In einem Beispiel wurde MEG mit Ammoniak bei 180°C und einem Druck von 300 bar in Gegenwart von Wasserstoff an einem Co-Katalysator umgesetzt. Die Katalysatoren werden durch Sintern, zweckmäßig oberhalb von 700°C hergestellt

In der WO 2007/093514 wird ein zweistufiges Verfahren zur Herstellung von EDA offenbart, wobei in der ersten Verfahrensstufe die Aminierung an einem Hydroaminierungskatalysator bis zu einem MEA-Umsatz von maximal 40% durchgeführt wird und in der zweiten Verfahrensstufe ein geträgerter Ru/Co-Katalysatorformkörper mit kleiner Geometrie eingesetzt wird und die zweite Stufe bei einer um mindestens 10°C höheren Temperatur als die erste Verfahrensstufe durchgeführt wird. Die Katalysatoren werden bei 200 bis 500°C calciniert. In den Beispielen erfolgt die Calcinierung in Gegenwart von Luft.

Die WO 2013072289 offenbart die Umsetzung von Alkoholen mit einer stickstoffhaltigen Verbindung an Katalysatoren, die neben Al, Cu, Ni und Co das Element Sn beinhalten. Als bevorzugte Alkohole werden Ethylenglykol und Monoethanolamin genannt. Die Calcinierung erfolgt im Allgemeinen bei Temperaturen im Bereich von 300 bis 800°C. In einem Beispiel erfolgt die Kalzinierung unter Durchleiten von Luft.

Katalysatoren für die Aminierung von Alkoholen, die Sn enthalten, werden ebenfalls in der WO 2011067200 offenbart. Sie dort beschriebenen Katalysatoren enthalten neben Sn auch noch die Elemente Co, Ni, Al und Cu. Die Calcinierung erfolgt im Allgemeinen bei Temperaturen im Bereich von 300 bis 800°C

Weitere Katalysatoren für die Aminierung von Alkoholen werden in der WO 200908051, der WO 2009080508, der WO 200006749 und der WO 20008006750 offenbart. Die Katalysatoren enthalten neben Zr und Ni auch Cu, Sn, Co und/oder Fe. Weitere Bestandteile sind Elemente wie V, Nb, S, O, La, B, W, Pb, Sb, Bi und In.

Die WO 96/38226 offenbart Katalysatoren für die Aminierung von Alkoholen, die Re, Ni, Co, B, Cu und/oder Ru enthalten. In einem Beispiel wird ein Träger aus SiO2 mit einer Lösung von NH4ReO4, Ni-Nitrat, H3BO3, Co-Nitrat und Cu-Nitrat getränkt und anschließend calciniert. In einem weiteren Tränkungsschritt wird der calcinierte und imprägnierte Träger mit Ru-Chlorid getränkt. Vor der Reduktion wird optional eine Calcinierung im Bereich von 200 bis 500°C durchgeführt, wobei die Calcinierung offenbarungsgemäß bevorzugt in Gegenwart von Luft erfolgt.

In der US 4,701,434 und der US 4,855,505 wird die Aminierung von MEG und MEA in Gegenwart von Katalysatoren offenbart, die Ni und/oder Co und Ru enthalten. Dabei wird ein Katalysatorvorläufer, der Ni- und/der Co-Oxid enthält mit einem Ru-Halogenid, beispielsweise Ru-Chlorid, in Kontakt gebracht und anschließend im Wasserstoffstom reduziert. Die Calcinierung der Katalysatorvorläufer erfolgt offenbarungsgemäß bei 300 bis 600°C in einem Luftstrom. Die anschließende Reduktion des mit Ru-Halogenid behandelten Katalysatorvorläufers erfolgt in zwei Stufen, wobei zunächst bei 150 bis 300°C reduziert wird und in einer zweiten Stufe die Temperatur auf 300 bis 600°C erhöht wird.

Die EP 0839 575 offenbart Katalysatoren, die Co, Ni und deren Gemische und Ru auf einem porösen Metalloxidträger enthalten. Die Herstellung der Katalysatoren erfolgt durch Imprägnierung des Trägers mit den Metallen, Trocken und Calcinieren des imprägnierten Trägers und Reduktion des calcinierten Trägers in einem Wasserstoffstrom. Es wird weiterhin offenbart, dass die Imprägnierung des Trägers mit Metallverbindungen in beliebiger Reihenfolge erfolgen kann. In einem Beispiel wird ein Träger zuerst mit einer Lösung von Ni-, Co- und Cu-Nitraten imprägniert, anschließend calciniert und mit einer wässrigen Ru-Nitratlösung nachgetränkt und erneut bei 400°C calciniert.

Die US 5,958,825 offenbart Katalysatoren, enthaltend Ni und Co und Ru, die durch Tränkung eines Trägermaterials und anschließener Trocknung und Calcinierung des imprägnierten Katalysators hergestellt werden. In Beispiel 1 der US 5,958,825 erfolgt zunächst die Imprägnierung eines Aluminiumoxidträgers mit NiO, CoO und CuO und nach einer Calcinierung bei 400°C wurde der Katalysatorvoräufer mit einer Ru-Nitratlösung besprüht. Der so erhaltene Katalysatorvorläufer wurde getrocknet und bei 400°C calciniert.

Die Aufgabe der vorliegenden Erfindung bestand darin, einen heterogenen Katalysator für die Aminierung von MEG und/oder MEA in der Flüssigphase zu entwickeln, der eine ausreichende Aktivität und Selektivität bei der Umsetzung von MEG zu MEA und/oder EDA zeigt.

Insbesondere sollte die Bildung von Wertprodukten, d.h. solchen Ethanolaminen oder Ethylenaminen mit einer hohen kommerziellen Bedeutung, insbesondere MEA und EDA, begünstigt werden und die Bildung von zyklischen Ethylenaminen, insbesondere PIP, und höheren Ethanolaminen, insbesondere AEEA, gering gehalten werden, da für PIP bzw. AEEA der kommerzielle Bedarf geringer ist als für EDA und MEA.

Insbesondere sollte auch die Konzentration bestimmter unerwünschter Nebenprodukte, wie NMEDA, NEEDA und Ethylamin (EA), verringert werden. NMEDA hat eine Flüchtigkeit, die sich kaum von EDA unterscheidet, so dass die beiden Komponenten nur mit hohem Trennaufwand zu separieren sind. Somit wäre es vorteilhaft, wenn bereits bei der Produktion nur geringe Mengen NMEDA gebildet würden. Die üblichen Produktspezifikationen von EDA erfordern, dass weniger als 500 ppm NMEDA in EDA vorhanden sein sollen.

Weiterhin sollten die Katalysatoren auch eine hohe Aktivität aufweisen und einen hohen MEG-Umsatz ermöglichen, um eine gute Raum-Zeit-Ausbeute zu erzielen.

Insgesamt sollte somit ein gutes Eigenschaftsspektrum in Bezug auf Gesamtselektivität, Selektivitätsquotient und die Bildung von unerwünschten Nebenprodukten erreicht werden.

Die Aufgabe der vorliegenden Erfindung wurde gelöst durch ein Verfahren zur Herstellung von Alkanolaminen und/oder Ethylenaminen in der Flüssigphase, durch Umsetzung von Ethylenglykol und/oder Monoethanolamin mit Ammoniak in Gegenwart eines Aminierungskatalysators, der ein oder mehrere Aktivmetalle ausgewählt aus Sn und den Elementen der Gruppen 8, 9, 10 und 11 des Periodensystems der Elemente enthält, dadurch gekennzeichnet, dass der Aminierungskatalysator durch reduktive Calcinierung eines Katalysatorvorläufers erhalten wird, wobei die Herstellung des Katalysator die in Anspruch 1 genannten Schritte umfasst.

Überraschenderweise wurde festgestellt, dass Aminierungskatalysatoren, welche erfindungsgemäß reduktiv calciniert werden, eine hohe Selektivität für die linearen Aminierungsprodukte MEA und EDA bei der Umsetzung von MEG mit NH3 aufweisen, während die Selektivität für das zyklische Aminierungsprodukt PIP und das höhere Ethanolamin AEEA gering ist. Weiterhin wurde festgestellt, dass durch die in das erfindungsgemäße Verfahren eingesetzten Katalysatoren weniger unerwünschte Nebenprodukte, wie NMEDA, gebildet werden. Außerdem wurde festgestellt, dass die in das erfindungsgemäße Verfahren eingesetzten Aminierungskatalysatoren eine hohe Aktivität für die Umsetzung von MEG aufweisen und somit hohe Raum-Zeit-Ausbeuten bei der Umsetzung ermöglichen.

Bei der Herstellung der Aminierungskatalysatoren durch reduktive Calcinierung, die für den Einsatz in die erfindungsgemäße Reaktion geeignet sind, können bei der reduktiven Calcinierung der Katalysatorvorläufer Stickoxide entstehen, die explosionsfähige Gemische bilden können. Stickoxide können sich insbesondere dann bilden, wenn bei der Herstellung von Katalysatorvorläufern ein katalytisches oder nicht-katalytisches Trägermaterial mit Nitraten oder Nitrosylnitraten der Aktivmetalle bzw. Katalysatorzusatzelemente während der Herstellung in Kontakt gebracht wurde.

Es war dehalb auch Aufgabe dieser Offenbarung ein Verfahren zur Herstellung von Aminierungskatalysatoren bereit zu stellen, das hohen Sicherheitsstandards genügt.

Die Aufgabe wurde gelöst durch ein Verfahren zur Herstellung eines Aminierungskatalysators, der ein oder mehrere Aktivmetalle ausgewählt aus Sn und den Elementen der Gruppen 8, 9, 10 und 11 des Periodensystems der Elemente enthält, wobei der Aminierungskatalysator durch reduktive Calcinierung eines Katalysatorvorläufers erhalten wird, dadurch gekennzeichnet, dass der Reaktor, in dem die reduktive Calcinierung des Katalysatorvorläufers erfolgt, mit einer Denox-Anlage verbunden ist.

Nachfolgend und voranstehend werden folgende Abkürzungen verwendet:
- AEEA:: Aminoethylethanolamin
- AEP:: Aminoethylpiperazin
- DETA:: Diethylentriamin
- EA:: Ethylamin
- EDA:: Ethylendiamin
- EO:: Ethylenoxid
- EDC:: Ethylendichlorid
- HEP:: Hydroxyethylpiperazin
- NEEDA: N-Ethyleethylendiamin
- NMEDA:: N-Methylethylendiamin
- MEA:: Monoethanolamin
- MEG:: Monoethylenglykol
- PEHA:: Pentaethylenhexamin
- PIP:: Piperazin
- TEPA:: Tetraethylenepentamin
- TETA:: Triethylenetetramin

### Aminierungskatalysatoren

Das erfindungsgemäße Verfahren zur Herstellung von Alkanolaminen und Ethylenaminen durch Umsetzung von MEG und/oder MEA mit NH₃ erfolgt in Gegenwart von Aminierungskatalysatoren.

### Katalysatorvorläufer

Die Aminierungskatalysatoren werden durch reduktive Calcinierung von Katalysatorvorläufern erhalten.

### Aktive Masse

Die eingesetzten Katalysatorvorläufer enthalten eine aktive Masse.

Die aktive Masse der Katalysatorvorläufer enthält Aktivmetalle und optional ein oder mehrere Katalysatorzusatzelemente, sowie optional ein oder mehrere Trägermaterialien.

### Aktivmetalle

Die aktive Masse des Katalysatorvorläufers enthält ein oder mehrere Aktivmetalle ausgewählt aus der Gruppe bestehend aus Sn und den Elementen der Gruppen 8, 9, 10 und 11 des Periodensystems der Elemente.

Bevorzugt enthält die aktive Masse des Katalysatorvorläufers ein oder mehrere Aktivmetalle ausgewählt aus der Gruppe bestehend aus Fe, Ru, Co, Ni, Cu und Sn.

Ganz besonders bevorzugt enthält die aktive Masse des Katalysatorvorläufers ein oder mehrere Aktivmetalle ausgewählt aus der Gruppe bestehend aus Ru, Co, Ni, Cu und Sn.

In einer besonders bevorzugten Ausführungsform ist ein der ein oder mehreren Aktivmetalle Ru oder Co.

In einer ganz besonders bevorzugten Ausführungsform ist ein der ein oder mehreren Aktivmetalle Ru.

In weiteren besonders ganz bevorzugten Ausführungsformen enthält der Aminierungskatalysatoren eine der nachfolgend genannten Kombinationen der Aktivmetalle:
Ru und Co;
Ru und Sn;
Ru und Cu;
Ru und Ni;
Ru und Co und Sn;
Ru und Co und Cu;
Ru und Co und Ni;
Ru und Sn und Cu;
Ru und Sn und Ni;
Ru und Cu und Ni;
Ru und Co und Sn und Cu;
Ru und Co und Sn und Ni;
Ru und Co und Sn und Cu und Ni;
Co und Sn;
Co und Cu;
Co und Ni;
Co und Sn und Cu;
Co und Sn und Ni;
Co und Cu und Ni; oder
Co und Sn und Cu und Ni;

In einer ganz besonder bevorzugten Ausführungsform enthält der Aminierungskatalysatoren eine der nachfolgend genannten Kombinationen, die sowohl Ru als auch Co enthält;
Ru und Co;
Ru und Co und Sn;
Ru und Co und Cu;
Ru und Co und Ni;
Ru und Co und Sn und Cu;
Ru und Co und Sn und Ni;
Ru und Co und Sn und Cu und Ni.

### Katalysatorzusatzelemente

Die aktive Masse der in das erfindungsgemäße Verfahren eingesetzten Katalysatorvorläufer kann optional ein oder mehrere Katalysatorzusatzelemente umfassen.

Bei den Katalysatorzusatzelementen handelt es sich um Metalle oder Halbmetalle ausgewählt aus den Gruppen 1 bis 17 des Periodensystems (ausgenommen die Aktivmetalle), dem Element P und den Metallen der seltenen Erden.

### Bevorzugte Katalysatorzusatzelemente sind Zr, Al, Pb, Bi, Ce, Y, und Mn

Besonders bevorzugte Katalysatorzusatzelemente sind Zr, Al, Y und Mn Ganz besonders bevorzugte Katalysatorzusatzelemente sind Zr und Al.

### Katalytisch aktive Komponenten

Im Katalysatorvorläufer liegen die Aktivmetalle und die Katalysatorzusatzelemente im Allgemeinen in Form ihrer sauerstoffhaltigen Verbindungen vor, beispielsweise als Carbonate, Oxide, Mischoxide bzw. Hydroxide der Katalysatorzusatzelemente bzw. Aktivmetalle.

Die sauerstoffhaltigen Verbindungen der Aktivmetalle und der Katalysatorzusatzelemente werden nachfolgend als katalytische aktive Komponenten bezeichnet.

Der Begriff katalytisch aktive Komponenten soll aber nicht implizieren, dass diese Verbindungen an sich bereits katalytisch aktiv sind. Die katalytisch aktiven Komponenten weisen in der Regel erst nach erfolgter Reduktion des Katalysatorvorläufers eine katalytische Aktivität in der erfindungsgemäßen Umsetzung auf.

In der Regel werden die katalytisch aktiven Komponenten aus löslichen Verbindungen der Aktivmetalle bzw. der Katalysatorzusatzelemente oder ausgefällten Niederschläge der Aktimetalle bzw. der Katalysatorzusatzelemente durch eine Calcinierung in die katalytisch aktiven Komponenten umgewandelt, wobei die Umwandlung in der Regel durch Entwässerung und/oder Zersetzung erfolgt.

### Trägermaterialien

Die katalytisch aktive Masse kann weiterhin ein oder mehrere Trägermaterialien umfassen.

Im Rahmen der vorliegenden Erfindung wird zwischen katalytischen Trägermaterialien und konventionellen Trägermaterialien unterschieden.

### KonventionelleTrägermaterialien

Bei den konventionellen Trägermaterialien handelt es sich im Allgemeinen um Katalysatorzusatzelemente, die als Feststoff bei der Herstellung der Katalysatorvorläufer eingesetzt werden und auf die die löslichen Verbindungen der Aktivmetalle und/oder Katalysatorzusatzelemente aufgefällt werden oder die mit den löslichen Verbindungen der Aktivmetalle oder Katalysatorzusatelemente getränkt werden. In der Regel sind konventionelle Trägermaterialien Feststoffe mit einer hohen Oberfläche.

Als konventionelles Trägermaterial kann das Katalysatorzusatzelement Kohlenstoff, beispielsweise in Form von Graphit, Ruß und/oder Aktivkohle verwendet werden.

Bevorzugte konventionelle Trägermaterialien sind Oxide der Katalysatorzusatzelemente Al, Ti, Zn, Zr und Si oder Mischungen davon, beispielsweise Aluminiumoxid (gamma, delta, theta, alpha, kappa, chi oder Mischungen daraus), Titandioxid (Anatase, Rutil, Brookit oder Mischungen daraus), Zinkoxid, Zirkondioxid Siliziumdioxid (wie Silica, pyrogenes Siliziumdioxid, Kieselgel oder Silikate), Alumosilicate, Mineralien, wie Hydrotalcit, Chrysotil und Sepiolit.

Besonders bevorzugte Trägermaterialien sind Aluminiumoxid oder Zirkonoxid oder Gemische davon.

In einer besonders bevorzugten Ausführungsform ist das konventionelle Trägermaterial Aluminiumoxid; Zirkonoxid oder ein Gemisch davon mit einem mittleren Durchmesser der Partikel d₅₀ im Bereich von 50 bis 2000 µm, bevorzugt 100 bis 1000 µm und besonders bevorzugt 300 bis 700 µm. In einer besonders bevorzugten Ausführungsform liegt der mittlere Durchmesser d₅₀ der Partikeln im Bereich von 1 bis 500 µm, bevorzugt 3 bis 400 µm und besonders bevorzugt 5 bis 300 µm. In den bevorzugten Ausführungsbeispielen liegt die Standardabweichung des Teilchendurchmessers in der Regel im Bereich von 5 bis 200%, bevorzugt 10 bis 100% und insbesondere bevorzugt 20 bis 80% des mittleren Durchmessers d₅₀.

### Katalytische Trägermaterialien

In einer ganz besonders bevorzugten Ausführungsform ist das Trägermaterial ein katalytisches Trägermaterial.

Ein katalytisches Trägermaterial ist ein Feststoff, der ein oder mehrere Aktivmetalle enthält. Katalytische Trägermaterialien sind insbesondere Verbindungen, die wie nachfolgend beschrieben, durch selbst durch Mischfällung, Auffällung oder Tränkung hergestellt und anschließend im Allgemeinen durch Abtrennen, Waschen, Trocknen und Calcinierung aufgearbeitet wird und ggf. durch einen Formgebungsschritt in die unten genannte Form und Geometrie gebracht.

### Herstellung von katalytischen Trägermaterialien

Katalytische Trägermaterialien können nach bekannten Verfahren, z.B. durch Fällungsreaktionen (z.B. Mischfällung oder Auffällung) oder Tränkung, hergestellt werden.

Katalytische Trägermaterialien können über eine gemeinsame Fällung (Mischfällung) von löslichen Verbindungen der Aktivmetalle bzw. Katalysatorzusatzelemente mit einem Fällungsmittel hergestellt. Dazu wird in der Regel eine oder mehrere lösliche Verbindung der entsprechenden Aktivmetalle und ggf. eine oder mehrere lösliche Verbindungen der Katalysatorzusatzelemente in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Verbindung der Aktivmetalle, kommen üblicherweise die entsprechenden Metallsalze, wie die Nitrate oder Nitrosylnitrate, Chloride, ,Sulfate, Carboxylate, insbesondere der Acetate, oder Nitrate oder Nitrosylnitrate, der voranstehend genannten Metalle in Betracht.

Als lösliche Verbindungen der Katalysatorzusatzelemente werden in der Regel wasserlösliche Verbindungen der Katalysatorzusatzelemente, beispielsweise die wasserlöslichen Nitrate oder Nitrosylnitrate, Chloride, Sulfate, Carboxylate, insbesondere die Acetate oder Nitrate oder Nitrosylnitrate eingesetzt.

Katalytische Trägermaterialien können weiterhin durch Auffällung hergestellt werden.

Unter Auffällung wird eine Herstellmethode verstanden, bei der in der Regel ein oder mehrere Trägermaterialien, bevorzugt konventionelle Trägermaterialien, in einer Flüssigkeit suspendiert werden und nachfolgend lösliche Verbindungen der Aktivmetalle, wie lösliche Metallsalze der Aktivmetalle, und optional lösliche Verbindungen der Katalysatorzusatzelemente zugegeben werden, welche dann durch Zugabe eines Fällungsmittels auf das suspendierten Trägermaterial aufgefällt werden (z.B. beschrieben in EP-A2-1 106 600, Seite 4, und A. B. Stiles, Catalyst Manufacture, Marcel Dekker, Inc., 1983, Seite 15).

Als lösliche Verbindungen der Aktivmetalle bzw. Katalysatorzusatzelemente werden in der Regel wasserlösliche Verbindungen der Aktivmetalle bzw. Katalysatorzusatzelemente, beispielsweise die wasserlöslichen Nitrate oder Nitrosylnitrate, Chloride, Sulfate, Carboxylate, insbesondere die Acetate oder Nitrate oder Nitrosylnitrate eingesetzt.

Die Trägermaterialien, die bei der Auffällung verwendet werden, können beispielsweise in Form von Splitt, Pulvern oder Formkörpern, wie Strängen, Tabletten, Kugeln oder Ringen, eingesetzt werden. Es werden bevorzugt Trägermaterialien eingesetzt, die bereits die nachfolgend beschriebene bevorzugte Form und Geometrie der Formkörper aufweisen (siehe Abschnitt Form und Geometrie der Trägermaterialien).

Üblicherweise werden bei den Fällungsreaktionen die löslichen Verbindungen der Aktivmetalle bzw. Katalysatorzusatzelemente durch Zugabe eines Fällungsmittels als schwer- oder unlösliche, basische Salze gefällt.

Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.

Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100 °C, besonders 30 bis 90 °C, insbesondere bei 50 bis 70 °C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle bzw. Halbmetalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die katalytischen Trägermaterialien können auch durch Tränkung von Trägermaterialien mit löslichen Verbindungen der Aktivmetalle bzw. Katalysatorzusatzelemente hergestellt werden (Imprägnierung oder Tränkung).

Die Trägermaterialien, die bei der Tränkung verwendet werden, können beispielsweise in Form von Splitt, Pulvern oder Formkörpern, wie Strängen, Tabletten, Kugeln oder Ringen, eingesetzt werden. Es werden bevorzugt Trägermaterialien eingesetzt, die bereits die nachfolgend beschriebene bevorzugte Form und Geometrie der Formkörper aufweisen (siehe Abschnitt Form und Geometrie der Trägermaterialien).

Die Tränkung der obengenannten Trägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung eines Salzes der Aktivmetalle oder Katalysatorzusatzelemente in einer oder mehreren Tränkstufen.

Als Salze der Aktivmetalle bzw. der Katalysatorzusatzelemente kommen in der Regel wasserlösliche Salze, wie die Carbonate, Nitrate oder Nitrosylnitrate, Carboxylate, insbesondere die Nitrate oder Nitrosylnitrate, Acetate oder Chloride der entsprechenden Aktivmetalle bzw. Katalysatorzusatzelemente in Betracht, die sich in der Regel unter den Bedingungen der Calcinierung zumindest teilweise in die entsprechenden Oxide oder Mischoxide umwandeln.

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird oder das Trägermaterial mit der Tränklösung besprüht wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Salzen beaufschlagt werden soll.

Zur Aufbringung mehrerer Aktivmetalle und/oder Katalysatorzusatzelemente und/oder basischer Elemente auf das Trägermaterial, kann die Tränkung gleichzeitig mit allen Salzen oder in beliebiger Reihenfolge der einzelnen Salze nacheinander erfolgen.

### Aufarbeitung der katalytischen Trägermaterialien

Die nach diesen Tränkungsverfahren erhaltenen getränkten katalytische Trägermaterialien oder die nach den Fällungsverfahren erhaltenen Niederschläge werden üblicherweise verarbeitet, indem sie von der Flüssigkeit, in der die Tränkung bzw. Fällung durchgeführt wurde abgetrennt werden, gewaschen, getrocknet, calciniert und ggf. konditioniert und einem Formgebungsprozess unterzogen werden.

### Abtrennung und Waschen

Nach der Herstellung der katalytischen Trägermaterialen, werden die so erhaltenen Niederschläge oder getränkten, konventionellen Trägermaterialien in der Regel von der Flüssigkeit, in der die Herstellung der katalytischen Trägermaterialien erfolgte, abgetrennt und gewaschen. Verfahren zur Abtrennung und Waschen der katalytischen Trägermaterialen sind beispielsweise aus dem Artikel "Heterogenous Catalysis and Solid Catalysts, 2. Development and Types of Solid Catalysts", in Ullmann's Encyclopedia of Industrial Chemistry (DOI: 10.1002/14356007.o05_o02) bekannt.

Als Waschflüssigkeit wird in der Regel eine Flüssigkeit verwendet, in der das abgetrennte katalytischen Trägermaterial schlecht löslich ist, die aber ein gutes Lösungsmittel für am Katalysator anhaftende Verunreinigungen, beispielsweise Fällungsmittel, darstellt. Eine bevorzugte Waschflüssigkeit ist Wasser.

Bei der Batch-Herstellung erfolgt die Abtrennung in der Regel mit Rahmen-Filterpressen. Das Waschen des Filterrückstands mit Waschflüssigkeit kann hierbei durch Durchleiten der Waschflüssigkeit in Gegenstromrichtung zur Filtrationsrichtung erfolgen.

Bei der kontinuierlichen Herstellung erfolgt die Abtrennung in der Regel mit Drehtrommel-Vakuumfiltern. Das Waschen des Filterrückstands erfolgt üblicherweise dadurch, dass der Filterrückstand mit der Waschflüssigkeit besprüht wird.

Die Abtrennung des katalytischen Trägermaterialskann auch durch Zentrifugation erfolgen. In der Regel erfolgt das Waschen hierbei durch Zugabe von Waschflüssigkeit beim zentrifugieren.

### Trocknen

Das abgetrennte katalytische Trägermaterialen wird in der Regel getrocknet.

Verfahren zur Trocknung der katalytischen Trägermaterialen sind beispielsweise aus dem Artikel "Heterogenous Catalysis and Solid Catalysts, 2. Development and Types of Solid Catalysts", in Ullmann's Encyclopedia of Industrial Chemistry (DOI10.1002/14356007.o05_o02) bekannt. Die Trocknung erfolgt dabei bei Temperaturen im Bereich von bevorzugt 60 bis 200 °C, insbesondere von 80 bis 160 °C und besonders bevorzugt von 100 bis 140 °C, wobei die Trocknungsdauer bevorzugt bei 6 h oder mehr, beispielsweise im Bereich von 6 bis 24 h, liegt. Es sind jedoch in Abhängigkeit vom Feuchtigkeitsgehalt des zu trocknenden Materials auch kürzere Trockenzeiten wie beispielsweise ungefähr 1, 2, 3, 4, oder 5 h möglich.

Die Trocknung des abgetrennten und gewaschenen katalytischen Trägermaterialien kann beispielsweise in Kammeröfen, Trommeltrocknern, Drehrohröfen oder Bandtrocknern erfolgen. Die Trocknung der katalytischen Trägermaterialen kann auch durch Sprühtrocknung einer Suspension des katalytischen Trägermaterials erfolgen.

### Calcinierung

In der Regel werden die katalytischen Trägermaterialen im Anschluss an die Trocknung calciniert.

Während der Calcinierung werden thermisch labile Verbindungen der Aktivmetalle bzw. Katalysatorzusatzelemente, wie Carbonate, Hydrogencarbonate, Nitrate oder Nitrosylnitrate, Chloride, Carboxylate, Oxidhydratre oder Hydroxide, zumindest teilweise in die entsprechenden Oxide und/oder Mischoxide umgewandelt.

Die Calcinierung erfolgt in der Regel bei einer Temperatur im Bereich von 250 bis 1200 °C, bevorzugt 300 bis 1100°C und insbesondere von 500 bis 1000 °C.

Die Calcinierung kann unter jeder geeigneten Gasatmosphäre erfolgen, wobei Luft und/oder Luftgemische, wie Magerluft, bevorzugt sind. Das Calcinieren kann aber auch in Gegenwart von Wasserstoff, Stickstoff, Helium, Argon und/oder Dampf oder Gemischen hiervon erfolgen.

Die Calcinierung erfolgt in der Regel in einem Muffelofen, einem Drehrohrofen und/oder einem Bandkalzinierofen durchgeführt, wobei die Calcinierungsdauer bevorzugt bei 1 h oder mehr, besondersbevorzugt im Bereich von 1 bis 24 h und ganz besonders bevorzugt im Bereich von 2 bis 12 h liegt.

### Zusammensetzung von katalytischen Trägermaterialien

Die Zusammensetzung der katalytischen Trägermaterialien kann mittels bekannten Methoden der Elementaranalyse, beispielsweise der Atomabsorptionsspektrometrie (AAS), der Atomemissionsspektrometrie (AES), der Röntgenfluoreszenzanalyse (RFA) oder der ICP-OES (Inductively Coupled Plasma Optical Emission Spectrometry) gemessen werden.

Die Konzentrationsangaben (in Gew.-%) der katalytisch aktiven Komponenten erfolgt im Rahmen der vorliegenden Erfindung als entsprechendes Oxid.

Die Katalysatorzusatzelemente der Gruppe 1 (Alkalimetalle) werden als M₂O berechnet, beispielsweise Na₂O.

Die Katalysatorzusatzelemente der Gruppe 2 (Erdalkalimetalle) werden als MO berechnet, beispielsweise MgO oder CaO.

Die Katalysatorzusatzelemente der Gruppe 13 (Bor-Gruppe) werden M₂O₃ berechnet, beispielsweise B₂O₃ oder Al₂O₃.

In der Kohlenstoffgruppe (Gruppe 14) wird Si als SiO₂, Ge als GeO, Sn als SnO und Pb als PbO berechnet.

In der Stickstoffgruppe (Gruppe 15) wird P als H₃PO₄, As als As₂O₃, Sb als Sb₂O₃ und Bi als Bi₂O₃ berechnet.

Der der Gruppe der Chalkogene (Gruppe 16) wird Se als SeO₂ und Te als TeO₂ berechnet.

In der Scandiumgruppe (Gruppe 3) wird Sc als Sc₂O₃, Y als Y₂O₃ und La als La₂O₃ berechnet. In der Titangruppe (Gruppe 4) wird Ti als TiO₂, Zr als ZrO₂ und Hf als HfO₂ berechnet.

In der Vanadiumgruppe (Gruppe 5) wird V als V₂O₅, Nb als Nb₂O₅ und Ta als Ta₂O₅ berechnet. In der Chromgruppe (Gruppe 6) wird Cr als CrO₂, Mo als MoO₃ und W als WO₂ berechnet.

In der Mangangruppe (Gruppe 7) wird Mn als MnO₂ und Re als Re₂O₇ berechnet.

In der Eisengruppe (Gruppe 8) wird Fe als Fe₂O₃, Ru als RuO₂ und Os als OsO₄ berechnet.

In der Cobaltgruppe (Gruppe 9) wird Co als CoO, Rh als RhO₂ und Ir als IrO₂ berechnet.

In der Nickelgruppe (Gruppe 10) wird Ni als NiO, Pd als PdO und Pt als PtO berechnet.

In der Kupfergruppe (Gruppe 11) wird Cu als CuO, Ag als AgO und Au als Au₂O₃ berechnet.

In der Zinkgruppe (Gruppe 12) wird Zn als ZnO, Cd als CdO und Hg als HgO berechnet.

Die Konzentrationsangaben (in Gew.-%) der Komponenten des katalytischen Trägermaterials beziehen sich jeweils - falls nicht anders angegeben - auf die Gesamtmasse des katalytischen Trägermaterials nach der letzten Calcinierung.

Die Zusammensetzung der katalytischen Trägermaterialien ist im Allgemeinen abhängig von der nachfolgend beschriebenen Herstellungsmethode (Mischfällung oder Auffällung bzw. Tränkung).

Die katalytischen Trägermaterialien bestehen bevorzugt nur aus katalytisch aktiven Komponenten der Aktivmetalle oder Katalysatorzusatzelemente, beispielsweise in Form eines konventionellen Trägermaterials, und gegebenenfalls einem Verformungshilfsmittel (wie z. B. Graphit oder Stearinsäure), falls das katalytische Trägermaterial als Formkörper eingesetzt wird.

Der Anteil der katalytisch aktiven Komponenten der Aktivmetalle bzw. Katalysatorzusatzelemente bezogen auf die Gesamtmasse des katalytischen Trägermaterials beträgt üblicherweise 70 bis 100 Gew.-%, bevorzugt 80 bis 100 Gew.-%, mehr bevorzugt 90 bis 100 Gew.-%, noch mehr bevorzugt 95 Gew.-% bis 100 Gew.-% und besonders bevorzugt 97 Gew.-% bis 100 Gew.-%.

Katalytische Trägermaterialien, die durch Mischfällung hergestellt werden enthalten kein konventionelles Trägermaterial. Wenn die Fällung, wie nachfolgend beschrieben, in Gegenwart eines konventionellemTrägermaterials erfolgt, so wird die Herstellungsmethode für katalytische Trägermaterialein im Rahmen der vorliegenden Erfindung als Auffällung bezeichnet.

Katalytische Trägermaterialien, die durch Mischfällung hergestellt werden, enthalten in der Regel 1 bis 3, besonders bevorzugt 1 bis 2 und insbesondere bevorzugt 1 Aktivmetall(e).

Unabhängig von der Anzahl der in der aktiven Masse vorhanden Aktivmetalle, liegt bei katalytischen Trägermaterialien, die durch Mischfällung hergestellt werden, die Masse der katalytisch aktiven Komponenten der Aktivmetalle bevorzugt im Bereich von 1 bis 95 Gew.-%, besonders bevorzugt 10 bis 90 Gew.-%, ganz besonders bevorzugt 20 bis 85 Gew.-% und insbesondere bevorzugt 50 bis 80 Gew.-%, bezogen auf die Gesamtmasse des katalytischen Trägermaterials und wobei die katalytisch aktiven Komponenten als Oxid berechnet werden.

Katalytische Trägermaterialien, die durch Mischfällung hergestellt werden, enthalten in der Regel 1 bis 5, besonders bevorzugt 1 bis 4 und insbesondere bevorzugt 1 bis 3 verschiedene Katalysatorzusatzelemente.

Unabhängig von der Anzahl der in der aktiven Masse vorhanden Katalysatorzusatzelemente, liegt bei katalytischen Trägermaterialien, die durch Mischfällung hergestellt werden, die Masse der katalytisch aktiven Komponenten der Katalysatorzusatzelemente bevorzugt im Bereich von 1 bis 90 Gew.-%, besonders bevorzugt 5 bis 80 Gew.-% und ganz besonders bevorzugt 10 bis 60 Gew.-%, bezogen auf die Gesamtmasse des katalytischen Trägermaterials und wobei die katalytisch aktiven Komponenten als Oxid berechnet werden.

Katalytische Trägermaterialien, die durch Auffällung hergestellt werden enthalten in der Regel 5 bis 95 Gew.%, bevorzugt 10 bis 80 Gew.-% und besonders bevorzugt 15 bis 60 Gew.-% konventionelles Trägermaterial.

Katalytische Trägermaterialien, die durch Auffällung hergestellt werden, enthalten in der Regel 1 bis 5, besonders bevorzugt 1 bis 4 und insbesondere bevorzugt 1 bis 3 Aktivmetalle.

Unabhängig von der Anzahl der in der aktiven Masse vorhanden Aktivmetalle, liegt bei katalytische Trägermaterialien, die durch Auffällung hergestellt werden, die Masse der katalytisch aktiven Komponenten der Aktivmetalle bevorzugt im Bereich von 5 bis 90 Gew.-%, besonders bevorzugt 10 bis 70 Gew.-% und ganz besonders bevorzugt 15 bis 60 Gew.-%, bezogen auf die Gesamtmasse der katalytische Trägermaterialien und wobei die katalytisch aktiven Komponenten als Oxid berechnet werden.

Katalytische Trägermaterialien, die durch Auffällung hergestellt werden, enthalten in der Regel 1 bis 5, besonders bevorzugt 1 bis 4 und insbesondere bevorzugt 1 bis 3 verschiedene Katalysatorzusatzelemente.

Unabhängig von der Anzahl der in der aktiven Masse vorhanden Katalysatorzusatzelemente, liegt bei katalytische Trägermaterialien, die durch Auffällung hergestellt werden, die Masse der katalytisch aktiven Komponenten der Katalysatorzusatzelemente bevorzugt im Bereich von 1 bis 80 Gew.-%, besonders bevorzugt 5 bis 70 Gew.-% und ganz besonders bevorzugt 10 bis 50 Gew.-%, bezogen auf die Gesamtmasse der katalytische Trägermaterialien und wobei die katalytisch aktiven Komponenten als Oxid berechnet werden.

Katalytische Trägermaterialien, die durch Tränkung hergestellt werden enthalten in der Regel 50 bis 99 Gew.%, bevorzugt 75 bis 98 Gew.-% und besonders bevorzugt 90 bis 97 Gew.-% konventionelles Trägermaterial.

Katalytische Trägermaterialien, die durch Tränkung hergestellt werden, enthalten in der Regel 1 bis 5, besonders bevorzugt 1 bis 4 und insbesondere bevorzugt 1 bis 3 Aktivmetalle.

Unabhängig von der Anzahl der in der aktiven Masse vorhanden Aktivmetalle, liegt bei katalytische Trägermaterialien, die durch Tränkung hergestellt werden, die Masse der katalytisch aktiven Komponenten der Aktivmetalle bevorzugt im Bereich von 1 bis 50 Gew.-%, besonders bevorzugt 2 bis 25 Gew.-% und ganz besonders bevorzugt 3 bis 10 Gew.-%, bezogen auf die Gesamtmasse der katalytischen Trägermaterialien und wobei die katalytisch aktiven Komponenten als Oxid berechnet werden.

Katalytische Trägermaterialien, die durch Tränkung hergestellt werden, enthalten in der Regel 1 bis 4, besonders bevorzugt 1 bis 3 und insbesondere bevorzugt 1 bis 2 verschiedene Katalysatorzusatzelemente.

Unabhängig von der Anzahl der in der aktiven Masse vorhanden Katalysatorzusatzelemente, liegt bei katalytische Trägermaterialien, die durch Tränkung hergestellt werden, die Masse der katalytisch aktiven Komponenten der Katalysatorzusatzelemente bevorzugt im Bereich von 1 bis 50 Gew.-%, besonders bevorzugt 2 bis 25 Gew.-% und ganz besonders bevorzugt 3 bis 10 Gew.-%, bezogen auf die Gesamtmasse der katalytische Trägermaterialien und wobei die katalytisch aktiven Komponenten als Oxid berechnet werden.

### Bevorzugte Zusammensetzungen von katalytischen Trägermaterialien

Als katalytische Trägermaterialien werden insbesondere folgende Zusammensetzungen besonders bevorzugt eingesetzt:
I)
   In einer bevorzugten Ausführungsform werden als katalytische Trägermaterialen solche Zusammensetzungen eingesetzt, deren katalytisch aktive Masse katalytisch aktive Komponenten von Zr, Cu und Ni und ein oder mehrere katalytisch aktive Komponenten von Sb, Pb, Bi und In enthält. Solche Zusammensetzungen sind beispielsweise in der WO 2008/006749 offenbart.
   In einer besonders bevorzugten Variante dieser Ausführungsform wird eine Zusammensetzung eingesetzt, der
      10 bis 75 Gew.-%, bevorzugt 25 bis 65 Gew.-%, besonders bevorzugt 30 bis 55 Gew.-% katalytisch aktive Komponenten des Zirkoniums, berechnet als ZrO₂;
      1 bis 30 Gew.-%, bevorzugt 2 bis 25 Gew.-%, besonders bevorzugt 5 bis 15 Gew.-% katalytisch aktive Komponenten des Kupfers, berechnet als CuO,
      10 bis 70 Gew.-% bevorzugt 20 bis 60 Gew.-%, besonders bevorzugt 30 bis 50 Gew.-% katalytisch aktive Komponenten des Nickels, berechnet als NiO,
      0,1 bis 10 Gew.-%, besonders im Bereich von 0,2 bis 7 Gew.-%, weiter besonders im Bereich von 0,4 bis 5 Gew.-%, ganz besonders im Bereich von 2 bis 4,5 Gew.-%, katalytisch aktive Komponenten eines oder mehrerer Metalle, ausgewählt aus Sb, Pb, Bi und In, jeweils berechnet als Sb₂O₃, PbO, Bi₂O₃ bzw. In₂O₃. enthält.
II)
   In einer bevorzugten Ausführungsform werden als katalytisches Trägermatertial eine Zusammensetzung eingesetzt,, deren katalytisch aktive Masse katalytisch aktive Komponenten Zr, Cu, Ni und Co und ein oder mehrere katalytisch aktive Komponenten eines oder mehrerer Katalysatorzusatzelemente von Pb, Bi, Sn, Sb und In, enthält. Solche Zusammensetzungen sind beispielsweise in der WO 2008/006750 offenbart.
   In einer besonders bevorzugten Variante dieser Ausführungsform wird eine Zusammensetzung eingesetzt, die
      10 bis 75 Gew.-%, bevorzugt 25 bis 65 Gew.-%, besonders bevorzugt 30 bis 55 Gew.-% katalytisch aktive Komponenten des Zirkoniums, berechnet als ZrO₂,
      1 bis 30 Gew.-%, bevorzugt 2 bis 25 Gew.-%, besonders bevorzugt 5 bis 15 Gew.-% katalytisch aktive Komponenten des Kupfers, berechnet als CuO, und
      10 bis 70 Gew.-% bevorzugt 13 bis 40 Gew.-%, besonders bevorzugt 16 bis 35 Gew.-% katalytisch aktive Komponenten des Nickels, berechnet als NiO,
      10 bis 50 Gew.-%, bevorzugt 13 bis 40 Gew.-%, besonders bevorzugt 16 bis 35 Gew.% katalytisch aktive Komponenten des Kobalts, berechnet als CoO, und
      0, 1 bis 10 Gew.-%, besonders im Bereich von 0,2 bis 7 Gew.-%, weiter besonders im Bereich von 0,4 bis 5 Gew.-%, katalytisch aktive Komponenten eines oder mehrerer Metalle, ausgewählt aus Pb, Bi, Sn, Sb und In, jeweils berechnet als PbO, Bi₂O₃, SnO, Sb₂O₃ bzw. In₂O₃ enthält.
III)
   In einer weiteren bevorzugten Ausführungsform werden als katalytische Träger soche Zusammensetzungen eingesetzt, deren katalytisch aktive Masse katalytisch aktive Komponenten von Zr, Ni und Fe und im Bereich von 0,2 bis 5,5 Gew.-% von ein oder mehreren katalytisch aktive Komponenten von Sn, Pb, Bi, Mo, Sb und/oder P, jeweils berechnet als SnO, PbO, Bi₂O₃, MoO₃, Sb₂O₃ bzw.H₃PO₄, enthält. Solche Zusammensetzungen sind beispielsweise in der WO 2009/080506 offenbart.
   In einer besonders bevorzugten Variante dieser Ausführungsform wird eine Zusammensetzung eingesetzt, die
      20 bis 70 Gew.-% katalytisch aktive Komponenten des Zirkoniums, berechnet als ZrO₂,
      15 bis 60 Gew.-% katalytisch aktive Komponenten des Nickels, berechnet als NiO, und
      0,5 bis 14 Gew.-%, bevorzugt 1,0 bis 10 Gew.-%, besonders bevorzugt 1,5 bis 6 Gew.% katalytisch aktive Komponenten des Eisens, berechnet als Fe₂O₃, und
      0,2 bis 5,5 Gew.-%, bevorzugt 0,5 bis 4,5 Gew.-%, besonders bevorzugt 0,7 bis 3,5 Gew.-%, katalytisch aktive Komponenten des Zinns, Bleis, Bismuths, Molybdäns, Antimons und/oder Phosphors, jeweils berechnet als SnO, PbO, Bi₂O₃, MoO₃, Sb₂O₃ bzw. H₃PO₄, enthält.
IV)
   In einer weiteren bevorzugten Ausführungsform werden als katalytische Träger solche Zusammensetzungen eingesetzt, deren katalytisch aktive Masse katalytisch aktive Komponenten von Zr, Cu, Ni enthält und
   im Bereich von 0,2 bis 40 Gew.-% katalytisch aktive Komponenten des Kobalts, berechnet als CoO,
   im Bereich von 0, 1 bis 5 Gew.-% katalytisch aktive Komponenten des Eisens, berechnet als Fe₂O₃, und
   im Bereich von 0, 1 bis 5 Gew.-% katalytisch aktive Komponenten des Bleis, Zinns, Bismuths und/oder Antimons, jeweils berechnet als PbO, SnO, Bi₂O₃ bzw. Sb₂O₃, enthält.
   Solche Zusammensetzungen sind beispielsweise in der WO2009/080508 offenbart.
   In einer besonders bevorzugten Variante dieser Ausführungsform wird eine Zusammensetzung eingesetzt, die
      20 bis 85 Gew.-%, besonders 25 bis 70 Gew.-%, weiter besonders 30 bis 60 Gew.-% katalytisch aktive Komponenten des Zirkoniums, berechnet als ZrO₂,
      0,2 bis 25 Gew.-%, besonders 3 bis 20 Gew.-%, weiter besonders 5 bis 15 Gew.-%, katalytisch aktive Komponenten des Kupfers, berechnet als CuO,
      0,2 bis 45 Gew.-%, besonders 10 bis 40 Gew.-%, weiter besonders 25 bis 35 Gew.-%, katalytisch aktive Komponenten des Nickels, berechnet als NiO,
      0,2 bis 40 Gew.-%, bevorzugt 1 bis 25 Gew.-%, besonders bevorzugt 2 bis 10 Gew.-%, katalytisch aktive Komponenten des Kobalts, berechnet als CoO,
      0, 1 bis 5 Gew.-%, bevorzugt 0,2 bis 4 Gew.-%, besonders bevorzugt 0,5 bis 3 Gew.-%, katalytisch aktive Komponenten des Eisens, berechnet als Fe₂O₃, und
      0, 1 bis 5,0 Gew.-%, besonders 0,3 bis 4,5 Gew.-%, weiter besonders 0,5 bis 4 Gew.%, katalytisch aktive Komponenten des Bleis, Zinns, Bismuths und/oder Antimons, jeweils berechnet als PbO, SnO, Bi₂O₃ bzw. Sb₂O₃. enthält.
V)
   In einer weiteren bevorzugten Ausführungsform wird als katalytisches Trägermaterial eine Zusammensetzung eingesetzt, deren katalytisch aktive Masse katalytisch aktive Komponenten Zr, Cu und Ni, und
   im Bereich von 1,0 bis 5,0 Gew.-% katalytisch aktive Komponenten des Kobalts, berechnet als CoO, und
   im Bereich von 0,2 bis 5,0 Gew.-% katalytisch aktive Komponenten des Vanadiums, Niobs, Schwefels, Phosphors, Galliums, Bors, Wolframs, Bleis und/oder Antimons, jeweils berechnet als V₂O₅, Nb₂O₅, H₂SO₄, H₃PO₄, Ga₂O₃, B₂O₃, WO₃, PbO bzw. Sb₂O₃, enthält.
   Solche Zusammensetzungen werden beispielsweise in der WO2009/080508 offenbart.
   In einer besonders bevorzugten Variante dieser Ausführungsform wird eine Zusammensetzung eingesetzt, die
      46 bis 65 Gew.-%, besonders 47 bis 60 Gew.-%, weiter besonders 48 bis 58 Gew.-%, katalytisch aktive Komponenten des Zirkoniums, berechnet als ZrO₂,
      5,5 bis 18 Gew.-%, besonders 6 bis 16 Gew.-%, weiter besonders 7 bis 14 Gew.-%, katalytisch aktive Komponenten des Kupfers, berechnet als CuO,
      20 bis 45 Gew.-%, besonders 25 bis 40 Gew.-%, weiter besonders 30 bis 39 Gew.-%, katalytisch aktive Komponenten des Nickels, berechnet als NiO,
      1,0 bis 5,0 Gew.-%, besonders im Bereich von 1,5 bis 4,5 Gew.-%, weiter besonders im Bereich von 2,0 bis 4,0 Gew.-%, katalytisch aktive Komponenten des Kobalts, berechnet als CoO, und
      0,2 bis 5,0 Gew.-%, besonders 0,3 bis 4,0 Gew.-%, weiter besonders 0,5 bis 3,0 Gew.%, katalytisch aktive Komponenten des Vanadiums, Niobs, Schwefels, Phosphors, Galiums, Bors, Wolframs, Bleis und/oder Antimons, jeweils berechnet als V₂O₅, Nb2O₅, H₂S0₄, H₃PO₄, Ga₂O₃, B₂O₃, WO₃, PbO bzw. Sb₂0₃.
VI)
   In einer weiteren bevorzugten Ausführungsform wird als katalytisches Trägermaterial eine Zusammensetzung eingesetzt, deren katalytisch aktive Masse katalytisch aktive Komponenten von Al, Cu, Ni, Co und Sn und
   im Bereich von 0,2 bis 5,0 Gew.-% katalytisch aktive Komponenten des Yttriums, Lanthans, Cers und/oder Hafniums, jeweils berechnet als Y₂0₃, La₂O₃, Ce₂O₃ bzw. Hf₂O₃, enthält.
   Solche Zusammensetzungen werden beispielsweise in der WO 2011/067200 offenbart.
   In einer besonders bevorzugten Variante dieser Ausführungsform wird eine Zusammensetzung eingesetzt, die
      0,2 bis 5,0 Gew.-%, besonders im Bereich von 0,4 bis 4,0 Gew.-%, weiter besonders im Bereich von 0,6 bis 3,0 Gew.-%, weiter besonders bevorzugt im Bereich von 0,7 bis 2,5 Gew.-%, katalytisch aktive Komponenten des Zinns, berechnet als SnO,
      10 bis 30 Gew.-%, weiter besonders im Bereich von 12 bis 28 Gew.-%, ganz besonders 15 bis 25 Gew.-%, katalytisch aktive Komponenten des Kobalts, berechnet als CoO,
      15 bis 80 Gew.-%, besonders 30 bis 70 Gew.-%, weiter besonders 35 bis 65 Gew.-%, katalytisch aktive Komponenten des Aluminiums, berechnet als Al₂0₃,
      1 bis 20 Gew.-%, besonders 2 bis 18 Gew.-%, weiter besonders 5 bis 15 Gew.-%, katalytisch aktive Komponenten des Kupfers, berechnet als CuO, und
      5 bis 35 Gew.-%, besonders 10 bis 30 Gew.-%, weiter besonders 12 bis 28 Gew.-%, ganz besonders 15 bis 25 Gew.-%, katalytisch aktive Komponenten des Nickels, berechnet als NiO, 0,2 bis 5,0 Gew.-%, besonders im Bereich von 0,4 bis 4,0 Gew.-%, weiter besonders im Bereich von 0,6 bis 3,0 Gew.-%, weiter besonders bevorzugt im Bereich von 0,7 bis 2,5 Gew.-%, katalytisch aktive Komponenten des Yttriums, Lanthans, Cers und/oder Hafniums, jeweils berechnet als Y₂O₃, La₂O₃, Ce₂O₃ bzw. Hf₂O₃. enthält.
VII)
   In einer weiteren bevorzugten Ausführungsform wird als katalytisches Trägermaterial eine Zusammensetzung eingesetzt, die dadurch hergestellt werden, dass eine Lösung (L) enthaltend Zinnnitrat und mindestens einen Komplexbildner auf den Träger aufgebracht werden, wobei die Lösung (L) keinen Feststoff oder einen Feststoffanteil von maximal 0,5 Gew.-% bezogen auf die Gesamtmenge an gelösten Komponenten enthält und die Lösung (L) zusätzlich mindestens ein weiteres Nickelsalz, Cobaltsalz und/oder Kupfersalz enthält, besonders bevorzugt Nickelnitrat, Cobaltnitrat und/oder Kupfernitrat enthält.
   Solche Zusammensetzungen sind beispielsweise in der WO 2013/072289 offenbart.
   In einer bevorzugten Variante dieser Ausführungsform wird eine Zusammensetzung eingesetzt, die
      0,2 bis 5 Gew.-% katalytisch aktive Komponenten des Zinn, berechnet als SnO
      15 bis 80 Gew.-% katalytisch aktive Komponenten des Aluminium, berechnet als Al2O3
      1 bis 20 Gew.-% katalytisch aktive Komponenten des Kupfer, berechnet als CuO
      5 bis 35 Gew.-% katalytisch aktive Komponenten des Nickel, berechnet als NiO, und
      5 bis 35 Gew.-% katalytisch aktive Komponenten des Cobalt, berechnet als CoO enthält.

In einer ganz besonders bevorzugten Variante dieser Ausführungsform werden Zusammensetzungen mit der zuvor genannten Zusammensetzung dadurch erhalten, dass lösliche Verbindungen von Co und Sn auf ein fein dispergiertes Trägermaterial aufgefällt werden, wobei die lösliche Verbindung Sn-Nitrat ist und die Auffällung in Gegenwart eines Komplexbildners erfolgt. Die lösliche Verbindung von Co ist vorzugsweise Co-Nitrat.

Die Auffällung erfolgt weiterhin bevorzugt in Gegenwart mindestens einer weiteren löslichen Verbindung eines Katalysatorzusatzelements, vorzugsweise einer löslichen Verbindung von Cu und/oder Ni. Weiterhin bevorzugt werden die Katalysatorzusatzelemente ebenfalls in Form ihrer Nitrate oder Nitrosylnitrate eingesetzt.

Der Komplexbildner wird bevorzugt ausgewählt aus der Gruppe bestehend aus Glykolsäure, Milchsäure, Hydracylsäure, Hydroxybuttersäure, Hydroxyvaleriansäure, Äpfelsäure, Mandelsäure, Citronensäure, Zuckersäuren, Tartronsäure, Weinsäure, Oxalsäurde,Malonsäure, Maleinsäure, Bernsteinsäure, Glutarsäure, Adipinsäure, Glycin, Hippursäure, EDTA, Alanin, Valin, Leucin oder Isoleucin.

Das Trägermaterial ist bevorzugt Aluminiumoxid oder Zirkonoxid oder ein Gemisch davon.

### Form und Geometrie der eingesetzten Trägermaterialien

Das Trägermaterial werden bevorzugt in Form von Pulver oder Splitt oder in Form von Formkörpern eingesetzt werden.

Wird das Trägermaterial als Pulver bzw. Splitt eingesetzt, so liegt der mittlere Durchmesser der Partikel d₅₀ in der Regel im Bereich von 50 bis 2000 µm, bevorzugt 100 bis 1000 µm und besonders bevorzugt 300 bis 700 µm. Die Standardabweichung des Teilchendurchmessers liegt in der Regel im Bereich von 5 bis 200%, bevorzugt 10 bis 100% und insbesondere bevorzugt 20 bis 80% des mittleren Durchmessers d₅₀

In einer besonders bevorzugten Ausführungsform liegt der mittlere Durchmesser d₅₀ der Partikeln des eingesetzten Pulvers oder Splitts im Bereich von 1 bis 500 µm, bevorzugt 3 bis 400 µm und besonders bevorzugt 5 bis 300 µm. Die Standardabweichung des Teilchendurchmessers liegt in der Regel im Bereich von 5 bis 200%, bevorzugt 10 bis 100% und insbesondere bevorzugt 20 bis 80% des mittleren Durchmessers d₅₀.

Die Trägermaterialien können jedoch auch bevorzugt in Form von Formkörpern in das erfindungsgemäße Verfahren eingesetzt.

Als Formkörper eignen sich Formkörper mit beliebiger Geometrie bzw. Form. Bevorzugte Formen sind Tabletten, Ringe, Zylinder, Sternstränge, Wagenräder oder Kugeln, besonders bevorzugt sind Tabletten, Ringe, Zylinder, Kugeln oder Sternstränge. Ganz besonders bevorzugt ist die Zylinderform

Bei Kugeln beträgt der Durchmesser der Kugelform bevorzugt 20 mm oder weniger, besonders bevorzugt 10 mm oder weniger, ganz besonders bevorzugt 5 mm oder weniger und insbesondere bevorzugt 3 mm oder weniger.

In einer bevorzugten Ausführungsform liegt bei Kugeln der Durchmesser der Kugelform bevorzugt im Bereich von 0,1 bis 20, besonders bevorzugt 0,5 bis 10 mm, ganz besonders bevorzugt 1 bis 5 mm und insbesondere besonders bevorzugt 1,5 bis 3 mm.

Bei Strängen oder Zylindern liegt das Verhältnis von Länge : Durchmessers bevorzugt im Bereich von 1:1 bis 20:1, besonders bevorzugt 1:1 bis 14:1, ganz besonders bevorzugt im Bereich von 1:1 bis 10:1 und insbesondere bevorzugt im Bereich von 1:2 bis 6:1.

Der Durchmesser der Stränge oder Zylinder beträgt bevorzugt 20 mm oder weniger, besonders bevorzugt 15 mm oder weniger, ganz besonders bevorzugt 10 mm oder weniger und insbesondere bevorzugt 3 mm oder weniger.

In einer bevorzugten Ausführungsform liegt der Durchmesser der Stränge oder Zylinder vorzugsweise im Bereich von 0,5 bis 20 mm, besonders bevorzugt im Bereich von 1 bis 15 mm, ganz besonders bevorzugt im Bereich von 1,5 bis 10 mm.

Bei Tabletten beträgt die Höhe h der Tablette vorzugsweise 20 mm oder weniger, besonders bevorzugt 10 mm oder weniger, ganz besonders bevorzugt 5 mm oder weniger und insbesondere bevorzugt 3 mm oder weniger.

In einer bevorzugten Ausführungsform liegt die Höhe h der Tablette bevorzugt im Bereich von 0,1 bis 20 mm, besonders bevorzugt im Bereich von 0,5 bis 15 mm, ganz besonders bevorzugt im Bereich von 1 bis 10 mm und insbesondere bevorzugt im Bereich von 1,5 bis 3 mm.

Das Verhältnis von Höhe h (bzw. Dicke) der Tablette zum Durchmesser D der Tablette beträgt bevorzugt 1:1 bis 1:5, besonders bevorzugt 1:1 bis 1:2,5 und ganz besonders bevorzugt 1:1 bis 1:2.

Der eingesetzte Formkörper weist bevorzugt eine Schüttdichte (nach EN ISO 6) im Bereich von 0,1 bis 3 kg/l, bevorzugt von 1,0 bis 2,5 kg/l und insbesondere bevorzugt 1,2 bis 1,8 kg/l auf.

### Formgebung

Bevorzugt werden Trägermaterialien eingesetzt, die bereits die zu vor beschriebene bevorzugte Form und Geometrie aufweisen.

Trägermaterialien, insbesondere katalytische Trägermaterialien, die nach ihrer Herstellung nicht die gewünschte Form und Geometrie aufweisen, können einem Formgebungsschritt unterzogen werden.

Im Rahmen der Formgebung werden die Trägermaterialien in der Regel konditioniert, in dem sie durch Vermahlen auf eine bestimmte Korngröße eingestellt werden.

Nach der Vermahlung kann das konditionierte Trägermaterial it weiteren Additiven, wie Formhilfsmitteln, beispielsweise Graphit, Bindemitteln, Porenbildner und Anteigmitteln vermischt werden und zu Formkörpern weiterverarbeitet werden. Vorzugsweise wird Trägermaterial nur mit Graphit als Formhilfsmittel vermischt und keine weiteren Additive bei der Formgebung zugegeben.

Gängige Verfahren der Formgebung sind beispielsweise im Ullmann [Ullmann's Encyclopedia Electronic Release 2000, Kapitel: "Catalysis and Catalysts", Seiten 28-32] und von Ertl et al.

[Ertl, Knözinger, Weitkamp, Handbook of Heterogenoeous Catalysis, VCH Weinheim, 1997, Seiten 98 ff] beschrieben.

Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Durch den Prozess der Formgebung können Formkörper mit der oben genannten Geometrie erhalten werden.

Die Formgebung kann aber auch durch Sprühtrocknung einer Suspension des Trägermaterials erfolgen.

Nach der Konditionierung bzw. Formgebung erfolgt in der Regel eine Temperung.

Die Temperung erfolgt in der Regel bei einer Temperatur im Bereich von 250 bis 1200 °C, bevorzugt 300 bis 1100°C und insbesondere von 500 bis 1000 °C.

Die Temperung kann unter jeder geeigneten Gasatmosphäre erfolgen. Bevorzugt erfolgt die Temperung in Gegenwart von Luft, wobei der Volumenanteil von Luft bevorzugt im Bereich von 20 bis 100, besonders bevorzugt 35 bis 90 und inbesondere bevorzugt 30 bis 70 Vol-% beträgt Die Temperung erfolgt in der Regel in einem Muffelofen, einem Drehrohrofen und/oder einem Bandkalzinierofen durchgeführt, wobei die Dauer bevorzugt bei 1 h oder mehr, besonders bevorzugt im Bereich von 1 bis 24 h und ganz besonders bevorzugt im Bereich von 2 bis 12 h liegt.

### Herstellung der Katalysatorvorläufer (Tränkung von konventionellen oder katalytischen Trägermaterialien)

Die Herstellung der Katalysatorvorläufer erfolgt bevorzugt dadaurch, dass konventionelles oder katalytisches Trägermaterial mit ein oder mehreren löslichen Verbindungen der Aktivmetalle und optional ein oder mehreren löslichen Verbindungen der. Katalysatorzusatzelemente in Kontakt gebracht wird, wobei das in Kontaktbringen bevozugt durch Tränkung oder Imprägnierung erfolgt.

Das Inkontaktbringen der Trägermaterialien mit den löslichen Verbindungen der Aktivmetalle bzw. Katalysatorzusatzelemente mit denTrägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung eines Salzes der Aktivmetalle oder Katalysatorzusatzelemente in einer oder mehreren Tränkstufen.

Als Salze der Aktivmetalle bzw. der Katalysatorzusatzelemente kommen in der Regel wasserlösliche Salze, wie die Carbonate, Nitrate oder Nitrosylnitrate, Carboxylate, insbesondere Acetate, bevorzugt die die Nitrate oder Nitrosylnitrate und Acetate und ganz besonders bevorzugt die Nitrate oder Nitrosylnitrate der entsprechenden Aktivmetalle bzw. Katalysatorzusatzelemente in Betracht, die sich in der Regel unter den Bedingungen der Calcinierung zumindest teilweise in die entsprechenden Oxide oder Mischoxide umwandeln.

Das Inkontaktbringen kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird oder das Trägermaterial mit der Tränklösung besprüht wird. Die Tränkung kann aber auch in überstehender Lösung erfolgen.

Bei mehrstufigen Tränkverfahren ist es zweckmäßig, zwischen einzelnen Tränkschritten zu trocknen. Die mehrstufige Tränkung ist vorteilhaft dann anzuwenden, wenn das Trägermaterial in größerer Menge mit Salzen beaufschlagt werden soll.

Zur Aufbringung mehrerer Aktivmetalle und/oder Katalysatorzusatzelemente und/oder basischer Elemente auf das Trägermaterial, kann die Tränkung gleichzeitig mit allen Salzen oder in beliebiger Reihenfolge der einzelnen Salze nacheinander erfolgen.

Bevorzugte konventionelle Trägermaterialien sind Trägermaterialien, die die Katalysatorzusatzelemente Al und Zr oder Gemische davon enthalten.

In einer ganz besonders bevorzugten Ausführungsform werden die löslichen Verbindungen der Aktivmetalle, die mit den Trägermaterial in Kontakt gebracht werden, teilweise oder vollständig in Form ihrer Nitrate oder Nitrosylnitrate eingesetzt werden. Ganz besonders bevorzugt werden als lösliche Verbindungen der Aktivmetalle ausschließlich die Nitrate oder Nitrosylnitrate der Aktivmetalle eingesetzt.

Es ist weiterhin bevorzugt, dass die löslichen Verbindungen der Katalysatorzusatzelemente, teilweise oder vollständig in Form ihrer Nitrate oder Nitrosylnitrate eingesetzt werden. Ganz besonders bevorzugt werden als lösliche Verbindungen der Katalysatorzusatzelemente ausschließlich die Nitrate oder Nitrosylnitrate der Katalysatorzusatzelemente eingesetzt.

In einer insbesonders bevorzugten Ausführungsform werden als lösliche Verbindungen der Aktivmetalle und Katalysatorzusatzelemente, mit denen das Trägermaterial in Kontakt gebracht wird, ausschließlich die entsprechenden Nitrate oder Nitrosylnitrate eingesetzt.

In einer weiteren bevorzugten Ausführungsform werden als Trägermaterialien katalytische Trägermaterialien eingesetzt, die als Aktivmetalle ein oder mehrere Aktivmetalle ausgewählt aus der Gruppe bestehend aus Ru, Co, Sn, Cu und Ni enthalten. Besonders bevorzugt werden als Trägermaterialien katalytische Trägermaterialien eingesetzt, die als Aktivmetalle ein oder mehrere Aktivmetalle ausgewählt aus der Gruppe bestehend aus Co, Sn, Cu und Ni enthalten.

Es ist weiterhin bevorzugt, dass die katalytischen Trägermaterialien ein oder mehrere Katalystorzusatzelemente ausgewählt aus der Gruppe bestehend aus Zr und AI enthalten.

Es ist ganz besonders bevorzugt als katalytische Trägermaterialien, die zuvor genannten bevorzugten Zusammensetzungen, einzusetzen.

Die Trägermaterialien können beispielsweise in Form von Splitt, Pulvern oder Formkörpern, wie Strängen, Tabletten, Kugeln oder Ringen, eingesetzt werden.

Es werden bevorzugt Trägermaterialien eingesetzt, die bereits die zuvor beschriebene bevorzugte Form und Geometrie der Formkörper aufweisen (siehe Abschnitt Form und Geometrie der Trägermaterialien).

Der Gehalt an löslichen Verbindungen der Aktivmetalle mit der das Trägermaterial in Kontakt gebracht wird, liegt pro Aktivmetall bevorzugt im Bereich von 0,1 bis 50 Gew.-%, bevorzugt 1 bis 40 Gew.-% und besonders bevorzugt 2 bis 15 Gew.-%.

In einer besonders bevorzugten Ausführungsform ist mindestens ein Aktivmetall, mit der das Trägermaterial getränkt wird Ru. Es ist weiterhin bevorzugt, dass in dieser Ausführungsform Ru in Form von Ru-Nitrosylnitrat eingesetzt wird.

In einer weiteren ganz besonders bevorzugten Ausführungsform werden Trägermaterialien mit Ru und Co getränkt, in dem das Trägermaterial gleichzeitig oder nacheinander mit einer löslichen Ru-Verbindung und einer löslichen Co-Verbindung in Kontakt gebracht werden, wobei sowohl Ru als auch Co bevorzugt in Form ihrer Nitrate oder Nitrosylnitrate eingesetzt werden.

Der Ru-Gehalt der Lösungen, mit der das Trägermaterial in Kontakt gebracht wird, liegt üblicherweise im Bereich von 0,1 bis 50 Gew.-%, bevorzugt 1 bis 40 Gew.-% und besonders bevorzugt 2 bis 15 Gew.-%.

Der Co-Gehalt der Lösungen, mit der das Trägermaterial in Kontakt gebracht wird, liegt üblicherweise im Bereich von 0,1 bis 20 Gew.-%, bevorzugt 0,1 bis 5 Gew.-% und besonders bevorzugt 0,15 bis 2 Gew.-%.

Durch das Inkontaktbringen des Trägermaterials mit den löslichen Verbindungen von Co und Ru
wird der Anteil von Ru im Katalysatorvorläufer um ca. 0,1 bis 5 Gew.-%, vorzugsweise 0,5 bis 4 Gew.-% und ganz besonders bevorzugt um 1 bis 3 Gew.-% erhöht und
der Anteil von Co im Katalysatorvorläufer um ca. 0,1 bis 5 Gew.-%, vorzugsweise 0,5 bis 3 Gew.-% und ganz besonders bevorzugt um 1 bis 2 Gew.-% erhöht, jeweils bezogen auf die Gesamtmasse des Katalysatorvorläufers.

Das Trägermaterial kann gleichzeitig oder nacheinander mit einer löslichen Ru-Verbindung und einer löslichen Co-Verbindung in Kontakt gebracht werden.

In einer bevorzugten Ausführungsform wird das Trägermaterial mit einer Lösung in Kontakt gebracht, die sowohl eine lösliche Verbindung von Ru als auch eine lösliche Verbindung von Co enthält.

In einer weiteren bevorzugten Ausführungsform wird das Trägermaterial in einer ersten Stufe mit einer Lösung in Kontakt gebracht, die eine lösliche Verbindung von Ru enthält und nachfolgend in einer zweiten Stufe mit einer Lösung in Kontakt gebracht, die eine lösliche Verbindungen von Co enthält.

In einer weiteren bevorzugten Ausführungsform wird das Trägermaterial in einer ersten Stufe mit einer Lösung in Kontakt gebracht, die eine lösliche Verbindung von Co enthält und nachfolgend in einer zweiten Stufe mit einer Lösung in Kontakt gebracht, die eine lösliche Verbindungen von Ru enthält.

Bei mehrstufigen Tränkverfahren kann zwischen den einzelnen Tränkschritten das Trägermaterial von der Tränklösung, wie nachfolgend beschrieben, abgetrennt und getrocknet werden. Erfolgt das Inkontaktbringen mit einer löslichen Ru- und einer löslichen Co-Verbindung in zwei oder mehreren Tränkschritten, so ist es bevorzugt, dass die zweite Tränkung direkt im Anschluss an den Trocknungsschritt des ersten Tränkungsschrittes erfolgt, ohne dass zwischen der ersten und zweiten Tränkung eine Calcinierung nach dem Trocknungsschritt erfolgt.

In dieser Ausführungsform ist das Trägermaterial bevorzugt Aluminiumoxid, Zirkonoxid oder ein Gemisch hiervon.

Insbesondere ist in dieser Ausführungsform auch bevorzugt, dass das Trägermaterial ein katalytisches Trägermaterial ist.

### Aufarbeitung der Katalysatorvorläufer

Die nach diesen Tränkungsverfahren erhaltenen getränkten Katalysatorvorläufer werden üblicherweise verarbeitet, indem sie von der Flüssigkeit, in der die Tränkung durchgeführt wurde abgetrennt, gewaschen und getrocknet werden.

Abtrennung und Waschen:
Die getränkten Katalysatorvorläufer werden in der Regel von der Flüssigkeit, in der die Herstellung der Katalysatorvorläufer erfolgte, abgetrennt und gewaschen.

Verfahren zur Abtrennung und Waschen der Katalysatorformläufer sind beispielsweise aus dem Artikel "Heterogenous Catalysis and Solid Catalysts, 2. Development and Types of Solid Catalysts", in Ullmann's Encyclopedia of Industrial Chemistry (DOI: 10.1002/14356007.o05_o02) bekannt.

Als Waschflüssigkeit wird in der Regel eine Flüssigkeit verwendet, in der der abgetrennte Katalysatorvorläufer schlecht löslich ist, die aber ein gutes Lösungsmittel für am Katalysator anhaftende Verunreinigungen, beispielsweise Fällungsmittel, darstellt. Eine bevorzugte Waschflüssigkeit ist Wasser.

Bei der Batch-Herstellung erfolgt die Abtrennung in der Regel mit Rahmen-Filterpressen. Das Waschen des Filterrückstands mit Waschflüssigkeit kann hierbei durch Durchleiten der Waschflüssigkeit in Gegenstromrichtung zur Filtrationsrichtung erfolgen.

Bei der kontinuierlichen Herstellung erfolgt die Abtrennung in der Regel mit Drehtrommel-Vakuumfiltern. Das Waschen des Filterrückstands erfolgt üblicherweise dadurch, dass der Filterrückstand mit der Waschflüssigkeit besprüht wird.

Die Abtrennung des Katalysatorvorläufers kann auch durch Zentrifugation erfolgen. In der Regel erfolgt das Waschen hierbei durch Zugabe von Waschflüssigkeit beim zentrifugieren.

### Trocknen

Der abgetrennte Katalysatorvorläufer wird in der Regel getrocknet.

Verfahren zur Trocknung der Katalysatorvorläufer sind beispielsweise aus dem Artikel "Heterogenous Catalysis and Solid Catalysts, 2. Development and Types of Solid Catalysts", in Ullmann's Encyclopedia of Industrial Chemistry (DOI: 10.1002/14356007.o05_o02) bekannt. Die Trocknung erfolgt dabei bei Temperaturen im Bereich von bevorzugt 60 bis 200 °C, insbesondere von 80 bis 160 °C und besonders bevorzugt von 100 bis 140 °C, wobei die Trocknungsdauer bevorzugt bei 6 h oder mehr, beispielsweise im Bereich von 6 bis 24 h, liegt. Es sind jedoch in Abhängigkeit vom Feuchtigkeitsgehalt des zu trocknenden Materials auch kürzere Trockenzeiten wie beispielsweise ungefähr 1, 2, 3, 4, oder 5 h möglich.

Die Trocknung des abgetrennten und gewaschenen Katalysatorvorläufers kann beispielsweise in Kammeröfen, Trommeltrocknern, Drehrohröfen oder Bandtrocknern erfolgen.

Die Trocknung des Katalysatorvorläufers kann auch durch Sprühtrocknung einer Suspension des Katalysatorvorläufers erfolgen.

In einer bevorzugten Ausführungsform erfolgt die Trocknung bei Temperaturen von 300°C und weniger.

### Zusammensetzung der Katalysatorvorläufer

### Anteil der aktiven Masse

Die in das Verfahren eingesetzten Katalysatorvorläufer werden bevorzugt in Form von Katalysatorvorläufern eingesetzt, die nur aus katalytisch aktiver Masse und gegebenenfalls einem Verformungshilfsmittel (wie z. B. Graphit oder Stearinsäure), falls der Katalysatorvorläufer als Formkörper eingesetzt wird, bestehen.

Der Anteil der katalytisch aktiven Masse bezogen auf die Gesamtmasse des Katalysatorvorläufers beträgt üblicherweise 70 bis 100 Gew.-%, bevorzugt 80 bis 100 Gew.-%, mehr bevorzugt 90 bis 100 Gew.-%, noch mehr bevorzugt 95 Gew.-% bis 100 Gew.-% und besonders bevorzugt 97 Gew.-% bis 100 Gew.-%.

### Bestimmung der Zusammensetzung der Katalysatorvorläufer

Die Zusammensetzung der Katalysatorvorläufer kann mittels bekannten Methoden der Elementaranalyse, beispielsweise der Atomabsorptionsspektrometrie (AAS), der Atomemissionsspektrometrie (AES), der Röntgenfluoreszenzanalyse (RFA) oder der ICP-OES (Inductively Coupled Plasma Optical Emission Spectrometry) gemessen werden.

Die Konzentrationsangaben (in Gew.-%) der katalytisch aktiven Komponenten erfolgt im Rahmen der vorliegenden Erfindung als entsprechendes Oxid.

Die Katalysatorzusatzelemente der Gruppe 1 (Alkalimetalle) werden als M₂O berechnet, beispielsweise Na₂O.

Die Katalysatorzusatzelemente der Gruppe 2 (Erdalkalimetalle) werden als MO berechnet, beispielsweise MgO oder CaO.

Die Katalysatorzusatzelemente der Gruppe 13 (Bor-Gruppe) werden M₂O₃ berechnet, beispielsweise B₂O₃ oder Al₂O₃.

In der Kohlenstoffgruppe (Gruppe 14) wird Si als SiO₂, Ge als GeO, Sn als SnO und Pb als PbO berechnet.

In der Stickstoffgruppe (Gruppe 15) wird P als H₃PO₄, As als As₂O₃, Sb als Sb₂O₃ und Bi als Bi₂O₃ berechnet.

Der der Gruppe der Chalkogene (Gruppe 16) wird Se als SeO₂ und Te als TeO₂ berechnet.

In der Scandiumgruppe (Gruppe 3) wird Sc als Sc₂O₃, Y als Y₂O₃ und La als La₂O₃ berechnet.

In der Titangruppe (Gruppe 4) wird Ti als TiO₂, Zr als ZrO₂ und Hf als HfO₂ berechnet.

In der Vanadiumgruppe (Gruppe 5) wird V als V₂O₅, Nb als Nb₂O₅ und Ta als Ta₂O₅ berechnet.

In der Chromgruppe (Gruppe 6) wird Cr als CrO₂, Mo als MoO₃ und W als WO₂ berechnet.

In der Mangangruppe (Gruppe 7) wird Mn als MnO₂ und Re als Re₂O₇ berechnet.

In der Eisengruppe (Gruppe 8) wird Fe als Fe₂O₃, Ru als RuO₂ und Os als OsO₄ berechnet.

In der Cobaltgruppe (Gruppe 9) wird Co als CoO, Rh als RhO₂ und Ir als IrO₂ berechnet.

In der Nickelgruppe (Gruppe 10) wird Ni als NiO, Pd als PdO und Pt als PtO berechnet.

In der Kupfergruppe (Gruppe 11) wird Cu als CuO, Ag als AgO und Au als Au₂O₃ berechnet.

In der Zinkgruppe (Gruppe 12) wird Zn als ZnO, Cd als CdO und Hg als HgO berechnet.

Die Konzentrationsangaben (in Gew.-%) der katalytisch aktiven Komponenten des Katalysatorvorläufers beziehen sich jeweils - falls nicht anders angegeben - auf die Gesamtmasse des Katalysatorvorläufers nach dem letzten Trocknungsschritt vor der reduktiven Calcinierung.

### Zusammensetzung der Katalysatorvorläufer

Wenn das Trägermaterial kein katalytisches Trägermaterial ist, dann enthält der Katalysatorvorläufer, unabhängig von der Anzahl der eingesetzten Aktivmetalle und Katalysatorzusatzelemente, bevorzugt:
0,01 bis 20 Gew.-% Aktivmetalle; und
80 bis 99,99 Gew.-% Katalysatorzusatzelemente; und
besonders bevorzugt
0,1 bis 10 Gew.-% Aktivmetalle; und
90 bis 99,9 Gew.-% Katalysatorzusatzelemente; und
ganz besonders bevorzugt
1 bis 5 Gew.-% Aktivmetalle; und
95 bis 99 Gew.-% Katalysatorzusatzelemente

Wenn das Trägermaterial ein katalytisches Trägermaterial ist, dann enthält der Katalysatorvorläufer, unabhängig von der Anzahl der eingesetzten Aktivmetalle und Katalysatorzusatzelemente, bevorzugt:
5 bis 95 Gew.-% Aktivmetalle; und
5 bis 95Gew.-% Katalysatorzusatzelemente; und
besonders bevorzugt
10 bis 90 Gew.-% Aktivmetalle; und
10 bis 90 Gew.-% Katalysatorzusatzelemente; und
ganz besonders bevorzugt
50 bis 80 Gew.-% Aktivmetalle; und
20 bis 50 Gew.-% Katalysatorzusatzelemente

### Bevorzugte Katalysatorvorläuferzusammensetzungen

In einer bevorzugten Ausführungsform enthält der Katalysatorvoläufer:
0,01 bis 20 Gew.-%, besonders bevorzugt 0,1 bis 15 Gew.-% und insbesondere bevorzugt 1 bis 10 Gew.-% katalytisch aktive Komponenten von Ru, berechnet als RuO; und
1 bis 50 Gew.-%, besonders bevorzugt 10 bis 45 Gew.-% und insbesondere bevorzugt 20 bis 40 Gew.%- katalytisch aktive Komponenten von Co, berechnet als CoO; und
0,1 bis 5 Gew.-%, besonders bevorzugt 0,2 bis 4 Gew.-% und insbesondere bevorzugt 1 bis 3 Gew.-% katalytisch aktive Komponenten von Sn, berechnet als SnO.

In einer besonders bevorzugten Ausführungsform enthält der Katalysatorvorläufer:
(i) 0,2 bis 5 Gew.-% katalytisch aktive Komponenten von Sn, berechnet als SnO,
(ii) 1 bis 35 Gew-% katalytisch aktive Komponenten von Co, berechnet als CoO,
(iii) 10 bis 80 Gew.-% katalytisch aktive Komponenten von Al und/oder Zr, berechnet als Al₂O₃ bzw. ZrO₂;
(iv) 1 bis 35 Gew.-% katalytisch aktive Komponenten von Cu und/oder 1 bis 35 Gew.-% katalytisch aktive Komponenten von Ni, berechnet als CuO bzw. NiO; und
(v) 0,01 bis 20 Gew.-% katalytisch aktive Komponenten von Ru, berechnet als RuO.

In einer besonders bevorzugten Ausführungsform enthält der Katalysatorvorläufer:
(i) 0,2 bis 5 Gew.-% katalytisch aktive Komponenten von Sn, berechnet als SnO,
(ii) 5 bis 35 Gew-% katalytisch aktive Komponenten von Co, berechnet als CoO,
(iii) 15 bis 80 Gew.-% katalytisch aktive Komponenten von Al und/oder Zr, berechnet als Al₂O₃ bzw. ZrO₂;
(iv) 1 bis 20 Gew.-% katalytisch aktive Komponenten von Cu, berechnet als CuO,
(v) 5 bis 35 Gew.-% katalytisch aktive Komponenten von Ni, berechnet als NiO; und
(vi) 0,1 bis 20 Gew.-% katalytisch aktive Komponenten von Ru, berechnet als RuO.

### Reduktive Calcinierung

Im Allgemeinen erfolgt im Anschluss an die Trocknung die erfindungsgmäße reduktive Calcinierung der Katalysatorvorläufer.

Die reduktive Calcinierung wird in Gegenwart eines reduzierenden Gases, insbesondere Wasserstoff, durchgeführt.

Zusätzlich kann die reduktive Calcinierung in Gegenwart eines Inertgases, vorzugsweise Stickstoff, Helium oder Argon durchführt werden, wobei der Volumenanteil von reduzierendem Gas, insbesondere Wasserstoff, in Gemischen mit Inertgas vorzugweise im Bereich von 1 bis 50 Vol.-%, besonders bevorzugt im Bereich von 2,5 bis 40 Vol.-% und ganz besonders bevorzugt im Bereich von 5 bis 20 Gew.-% liegt.

Es ist weiterhin bevorzugt den Anteil von Wasserstoff im Gemisch mit Inertgas graduell oder stufenweise zu erhöhen, beispielsweise von 0 Vol.% Wasserstoff auf 20 Vol.% Wasserstoff. So kann beim Aufheizen der Volumenanteil von Wasserstoff 0 Vol.-% betragen und nach Erreichen der Calcinierungstemperatur in ein oder mehreren Stufen oder graduell auf 20 Vol-% erhöht werden.

Die Temperatur bei der reduktiven Calcinierung beträgt vorzugsweise 100 bis 400°C, besonders bevorzugt 150 bis 350°C, besonders bevorzugt 180 bis 300°C und ganz besonders bevorzugt 200 bis 280°C.

Insbesondere ist es bevorzugt, dass die Temperatur bei der reduktiven Calcinierung 300°C nicht übersteigt. In diesem Fall können werden Katalysatoren erhalten, die ein besonders positives Eigenschaftsprofil in Hinblick auf Selektivität, Aktivität und der Vermeidung unerwünschter Nebenprodukte liefern.

Im Anschluss an die reduktive Calcinierung erfolgt in der Regel eine Passivierung, beispielsweise, wie nachfolgend beschrieben.

Die reduktive Calcinierung erfolgt in der Regel in einem Muffelofen, einem Drehrohrofen, einem Schachtreaktor, einem Drehrohrofen, einem Etagenofen, einem Wirbelschichtreaktor und/oder einem Bandkalzinierofen.

Bevorzugt erfolgt die reduktive Calcinierung in einem Schachtreaktor oder Drehrohrofen.

Die Calcinierungsdauer bei der reduktiven Calcinierung liegt bevorzugt bei 1 h oder mehr, besonders bevorzugt im Bereich von 1 bis 24 h und ganz besonders bevorzugt im Bereich von 2 bis 12 h liegt.

Besonders bevorzugt ist der Reaktor, in dem die reduktive Calcinierung des Katalysatorvorläufers erfolgt, wie untenstehend beschrieben, mit einer Denox-Anlage verbunden.

### Denox-Anlage

Bei der Herstellung der Aminierungskatalysatoren durch reduktive Calcinierung können ggf. bei der reduktiven Calcinierung der Katalysatorvorläufer Stickoxide enstehen, welche explosionsfähige Gemische bilden können. Stickoxide können sich insbesondere dann bilden, wenn bei der Herstellung der Katalysatorvorläufer eine katalytisches oder nicht-katalytisches Trägermaterial mit den Nitraten oder Nitrosylnitraten der Aktivmetalle bzw. Katalysatorzusatzelemente in Kontakt gebracht wurde.

Es war dehalb auch Aufgabe dieser Offenbarung ein Verfahren zur Herstellung von Aminierungskatalysatoren bereit zu stellen, das hohen Sicherheitsstandards genügt.

Die Aufgabe wurde dadurch gelöst, dass eine Denox-Anlage bei der Herstellung von Aminierungskatalysatoren verwendet wurde.

Die Aufgabe wurde weiterhin gelöst durch ein Verfahren zur Herstellung von Aminierungskatalysatoren, der ein oder mehrere Elemente ausgewählt aus Sn und den Elementen der Gruppen 8, 9, 10 und 11 des Periodensystems der Elemente enthält, wobei der Aminierungskatalysator duch reduktive Calcinierung erhalten wird, dadurch gekennzeichnet, dass der Reaktor in dem die reduktive Calcinierung erfolgt, mit einer Denox-Anlage verbunden ist.

Bevorzugt erfolgen die Herstellung der Katalysatorvorläufer und die reduktive Calcinierung wie voranstehend bereits beschrieben, wobei die jeweils als bevorzugt genannten Varianten und Ausführungsformen auch bei der vorliegenden Herstellung der Aminierungskatalysatoren bevorzugt ist.

Insbesondere bevorzugt ist es das beschriebene Verfahren zur Herstellung von Aminierungskatalysatoren anzuwenden, wenn bei der Herstellung der Katalysatorvorläufer eine katalytisches oder nicht-katalytisches Trägermaterial mit ein oder mehreren Nitraten oder Nitrosylnitraten der Aktivmetalle bzw. Katalysatorzusatzelemente in Kontakt gebracht wurde.

In einer besonders bevorzugten Ausführungsform ist der Reaktor, in dem die reduktive Calcinierung erfolgt, mit einer Denox-Anlage verbunden. Dies hat den Vorteil, dass bei der reduktiven Calcinierung entstehende, ggf. explosive Stickoxide vernichtet werden können.

Die reduktive Calcinierung erfolgt in der Regel, wie voranstehend bereits beschrieben, in einem Muffelofen, einem Drehrohrofen, einem Schachtreaktor, einem Etagenofen, einem Wirbelschichtreaktor und/oder einem Bandkalzinierofen.

Bevorzugt erfolgt die reduktive Calcinierung in einem Schachtreaktor oder Drehrohrofen.

Der Reaktor, in dem die reduktive Calcinierung erfolgt, ist in einer besonderen Ausgestaltung der vorliegenden Offenbarung mit einer Denox-Anlage verbunden, so dass der Gasstrom vom Ausgang des Reaktors, in dem die reduktive Calcinierung durchgeführt wird, zum Eingang der Denox-Anlage geleitet wird.

In der Denox-Anlage werden im Allgemeinen die Stickoxide, die sich im Gasstrom befinden können, partiell oder vollständig vernichtet.

Die Denox-Anlage kann bevorzugt als Gaswäsche oder als selektive katalytische Reduktion ausgestaltet sein.

Bei der selektiven katalytischen Reduktion wird in der Regel Ammoniak dem Abgas aus der reduktiven Calcinierung als Reduktionsmittel zugemischt und an Denox-Katalysatoren, beispielsweise titanoxid- und vanadiumoxidhaltigen Katalysatoren, vorbeigeleitet. Hierbei werden die Stickoxide in der Regel mit Ammoniak zu Wasser, in Form von Wasserdampf, und Stickstoff umgesetzt.

In einer bevorzugten Ausführungsform wird der für die Reduktion erforderliche Ammoniak zusammen mit dem Wasserstoff zugeführt. Der Volumenanteil von Ammoniak im Gasstrom aus reduzierendem und ggf. inerten Gasen beträgt üblicherweise 5 bis 50 Vol.%, bevorzugt 10 bis 40 Vol.% und besonders bevorzugt 20 bis 30 Vol.%, jeweils bezogen auf den Gesamtgasstrom, der über den Katalysatorvorläufer bei der reduktiven Calcinierung geleitet wird.

In einer weiteren bevorzugten Ausführungsform wird Ammoniak erst hinter dem Reaktor, in dem die reduktive Calcinierung erfolgt, und vor der Denox-Anlage zugefügt.

Weniger bevorzugt ist es den Ammoniak als wässrige Harnstofflösung zuzuführen, da hierbei durch eine Hydrolysereaktion von Harnstoff Ammoniak und CO2 entsteht. Das entstehende CO2 kann als Katalysatorgift die Aktivität der Aminierungskatalysatoren vermindern.

Die Temperatur des Gasstromes, der über die Denox-Katalysatoren geleitet wird, beträgt vorzugsweise 100 bis 400°C, besonders bevorzugt 150 bis 350°C, besonders bevorzugt 180 bis 300°C und ganz besonders bevorzugt 200 bis 280°C. Dies entspricht im Wesentlichen der Temperatur, die auchbei der reduktiven Calcinierung, angewandt wird, so dass eine weitere Temperierung des Gasstromes vor dem Überleiten über den Denox-Katalysator in der Regel nicht erforderlich ist.

Das bei der Reduktion mit Ammoniak entstehende Wasser wird üblicherweise hinter den Denox-Katalysatoren aus dem Gasstrom entfernt, vozugsweise durch Auskondensation oder durch Trocknung mit geeigneten Molsieben und der Gasstrom in Kreisfahrweise, ggf. unter Beimengung von zusätzlichem Wasserstoff, in die reduktive Calcinierung zurückgeführt.

Die Denox-Anlage kann auch als Gaswäsche ausgestaltet sein. Hierbei wird im Allgemeinen der stickoxidhaltige Gasstrom mit einer Waschflüssigkeit in Kontakt gebracht. Als Waschflüssgkeiten kommen im Allgemeinen basische Substanzen, beispielsweise wässrige Suspensionen oder Lösungen von Alkalihydroxiden, Alkalicarbonaten, Erdalkalihydroxiden, Erdalkalicarbonaten, Ammoniak und Harnstoff in Betracht. Bevorzugt sind wässrige Lösungen von Alkalihydroxiden, insbesondere NaOH und KOH, wässrige Lösungen oder Suspensionen von Erdalkalicarbonaten oder Erdalkalihydroxiden, insbesondere Mg-Hydroxid, Mg-Carbonat, Ca-Hydroxid, Ca-Carbonat, wässrige Lösungen von Ammoniak oder wässrige Harnstofflösungen.

Als Waschflüssigkeit kann aber auch eine wässrige Wasserstoffperoxidlösung oder auch Wasser verwendet werden.

Das Inkontaktbringen des stickoxidhaltigen Gasstrom erfolgt bevorzugt in einem Absorber, der als Tauschwäscher, Sprühwäscher, Füllkörper- oder Bodenkolonne, Strahlwäscher, Wirbelwäscher, Rotationswäscher oder Venturiwäscher ausgestaltet sein kann. Vorzugsweise ist der Absorber als Füllkörper- oder Bodenkolonne oder Sprühwäscher ausgestaltet. Die Behandlung des Gasstromes mit der Waschflüssigkeit erfolgt dabei bevorzugt in einer Kolonne im Gegenstrom. Der Gasstrom wird dabei im Allgemeinen in den unteren Bereich und die Waschflüssigkeit in den oberen Bereich der Kolonne eingespeist. Gemäß einer bevorzugten Ausführungsform wird der Waschschritt so durchgeführt, dass der stickoxidhaltige Gasstrom in einem Waschschritt mit der Waschflüssigkeit bei einer Temperatur von 20 bis 80°C, bevorzugt 20 bis 70°C und insbesondere 30 bis 60°C behandelt wird. Der Gesamtdruck im Waschschritt entspricht in der Regel dem Druck, bei dem auch die reduktive Calcinierung durchgeführt wird, bevorzugt 1 bar abs. Die genauen Betriebsbedingungen der Wäsche können vom Fachmann routinemäßig ermittelt werden.

Die Waschflüssigkeit kann durch Membranverfahren, Erhitzen, Entspannen auf einen niedrigeren Druck oder Strippen regeneriert werden. Nach dem Regenerationsprozess kann die Waschflüssigkeit wiederverwendert werden.

Der Gasstrom, der die Denox-Analge verlässt, weist im Allgemeinen eine Konzentration von Stickoxiden auf, die geringer ist als die Konzentration, die vor der Denox-Anlage im Gasstrom vorhanden war. Der an Stickoxiden abgereicherte Gasstrom kann, ggf. unter Beimengen von Wasserstoff und/oder Inertgas, wieder in die Stufe der reduktiven Calcinierung zurückgeführt werden.

Im Anschluss an die reduktive Calcinierung erfolgt in der Regel eine Passivierung, beispielsweise, wie nachfolgend beschrieben.

### Passivierung

Der Katalysator wird nach der reduktiven Calcinierung bevorzugt mit einem Sauerstoff enthaltenden Gasstrom wie Luft oder einem Gemisch von Luft mit Stickstoff in Kontakt gebracht werden.

Dadurch wird ein passivierter Katalysator erhalten. Der passivierte Katalysator weist im Allgemeinen eine schützende Oxidschicht auf. Durch diese schützende Oxidschicht wird die Handhabung und Lagerung des Katalysators vereinfacht, so dass beispielsweise der Einbau des passivierten Katalysators in den Reaktor vereinfacht wird.

Zur Passivierung wird im Anschluss an die reduktive Calcinierung mit einem sauerstoffhaltigen Gas, bevorzugt Luft, in Kontakt gebracht wird. Das sauerstoffhaltige Gas kann in Beimengungen mit Inertgasen, wie Stickstoff, Helium, Neon, Argon oder Kohlendioxid zum Einsatz kommen. In einer bevorzugten Ausführungsform wird Luft zusammen mit Stickstoff eingesetzt, wobei der Volumenanteil von Luft bevorzugt im Bereich von 1 bis 80, besonders bevorzugt 20 bis 70 und insbesondere bevorzugt 30 bis 60 Vol-% beträgt. In einer bevorzugten Ausführungsform wird der Volumenanteil von Luft im Gemisch mit Stickstoff allmählich von 0 auf ungefähr 50 Vol.-% erhöht.

Die Passivierung erfolgt bevorzugt bei Temperaturen bis 50°C, bevorzugt bis 45°C und ganz besonders bevorzugt bis 35°C.

### Aktivierung

Ein passivierter Katalysator wird bevorzugt vor dem Inkontaktbringen mit den Edukten durch Behandlung des passivierten Katalysators mit Wasserstoff oder einem Wasserstoff enthaltenden Gas reduziert.

Durch die Aktivierung wird in der Regel die schützende Passivierungsschicht aufgehoben.

Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. In einer bevorzugten Ausführungsform wird Wasserstoff zusammen mit Stickstoff eingesetzt, wobei der Volumenanteil von Wasserstoff bevorzugt im Bereich von 1 bis 50, besonders bevorzugt 2,5 bis 30 und inbesondere bevorzugt 5 bis 25 Vol-% beträgt. Der Wasserstoffstrom kann auch als Kreisgas in die Reduktion zurückgeführt werden, ggf. vermischt mit Frisch-Wasserstoff und ggf. nach Entfernen von Wasser durch Kondensation.

Die Aktivierung erfolgt bevorzugt in einem bewegten oder unbewegten Reduktionsofen durchgeführt werden.

Die Aktivierung des Katalysatorvorläufers erfolgt besonders bevorzugt in einem Reaktor, in dem die Katalysatorvorläufer als Festbett angeordnet sind. Besonders bevorzugt erfolgt die Reduktion des Katalysatorvorläufers in demselben Reaktor in dem die nachfolgende Umsetzung von MEG und/oder MEA mit NH3 erfolgt.

Die Aktivierung des Katalysatorvorläufers erfolgt in der Regel bei Reduktionstemperaturen von 50 bis 600°C, insbesondere von 100 bis 500°C, besonders bevorzugt von 150 bis 450°C.

Der Wasserstoffpartialdruck beträgt in der Regel von 1 bis 300 bar, insbesondere von 1 bis 200 bar, besonders bevorzugt von 1 bis 100 bar, wobei sich die Druckangaben hier und im Folgenden auf den absolut gemessenen Druck beziehen.

In einer besonders bevorzugten Ausführungsform liegt die Temperatur bei der Aktivierung im Bereich, in dem auch die reduktive Calcinierung durchgeführt wurde, nämlich vorzugsweise bei 100 bis 400°C, besonders bevorzugt 150 bis 350°C, besonders bevorzugt 180 bis 300°C und ganz besonders bevorzugt 200 bis 280°C. Wenn die Aktivierung auch in diesem niedrigeren Temperaturbereich durchgeführt wird, werden Aminierungskatalysatoren mit einem besonders vorteilhaften Eigenschaftsprofil erhalten.

### Edukte

Die erfindungsgemäße Umsetzung von Ethylenglykol (EG) und/oder Monoethanolamin (MEA) und Ammoniak (NH₃) in Gegenwart der reduzierten bzw. aktivierten Aminierungskatalysatoren erfolgt erfindungsgemäß in der Flüssigphase.

### Ethylenglykol

Als Ethylenglykol wird bevorzugt technisches Ethylenglykol mit einer Reinheit von mindestens 98%, und ganz besonders bevorzugt Ethylenglykol mit einer Reinheit von mindestens 99% und ganz besonders bevorzugt von mindestens 99,5%.

Das in das Verfahren eingesetzte Ethylenglykol kann aus Ethylen hergestellt werden, welches aus petrochemischen Prozessen erhältlich ist. So wird in der Regel in einer ersten Stufe Ethen zu Ethylenoxid oxidiert, welches nachfolgend mit Wasser zu Ethylenglykol umgesetzt wird. Das erhaltene Ethylenoxid kann aber auch im sogenannten Omega-Prozess mit Carbondioxid zu Ethylencarbonat umgesetzt werden, welches anschließend mit Wasser zu Ethylenglykol zu hydrolisieren kann. Das Omega-Verfahren zeichnet sich durch eine höhere Selektivität für Ethylenglykol aus, da weniger Nebenprodukte, wie Di- und Triethylenglykol entstehen.

Ethen kann aber auch aus nachwachsen Rohstoffen hergestellt werden. So kann Ethen durch Dehydratisierung von Bio-Ethanol gebildet werden.

Ethylenglykol lässt sich auch über den Synthesegasweg, z.B. durch oxidative Carbonylierung von Methanol zu Dimethyloxalat und dessen anschließender Hydrierung, herstellen. Damit kommen als weiterer petrochemischer Rohstoff auch Erdgas oder Kohle für die Herstellung von MEG in Frage.

### MEA

In das erfindungsgemäße Verfahren kann auch MEA eingesetzt werden.

MEA kann, wie voranstehend beschrieben, durch Umsetzung von Ethylenoxid mit Ammoniak hergestellt werden.

Vorzugsweise kann MEA durch Umsetzung von MEG mit Ammoniak hergestellt werden, beispielsweise durch das erfindungsgemäße Verfahren, in dem zunächst MEG mit Ammoniak umgesetzt wird und das neben EDA entstehende MEA von EDA getrennt wird und das abgetrennte MEA, ggf. zusammen mit nicht umgesetzten MEG, in das erfindungsgemäße Herstellungsverfahren zurückführt wird.

Wenn MEA ohne MEG in das erfindungsgemäße Verfahren eingesetzt wird, so wird MEA bevorzugt mit einer Reinheit von mindestens 97%, und ganz besonders bevorzugt mit einer Reinheit von mindestens 98% und ganz besonders bevorzugt von mindestens 99% eingesetzt. Wenn MEA zusammen mit MEG in das erfindungsgemäße Verfahren eingesetzt wird, so liegt der Gewichtsanteil von MEA in Bezug auf die Masse von MEA und MEG bevorzugt im Bereich von 0 bis 60 Gew.-%, besonders bevorzugt 10 bis 50 Gew.-% und ganz besonders bevorzugt 20 bis 40 Gew.-%.

### Ammoniak

Erfindungsgemäß erfolgt die Umsetzung von Ethylenglykol und/oder Monoethanolamin mit Ammoniak.

Als Ammoniak kann herkömmlich im Handel erhältliches Ammoniak eingesetzt werden, beispielsweise Ammoniak mit einem Gehalt von mehr 98 Gew.-% Ammoniak, bevorzugt mehr als 99 Gew.-% Ammoniak, bevorzugt mehr als 99,5 Gew.-%, insbesondere mehr als 99,8 Gew.-% Ammoniak.

### Wasserstoff

Das erfindungsgemäße Verfahren erfolgt bevorzugt in Gegenwart von Wasserstoff.

Der Wasserstoff kommt im Allgemeinen technisch rein zum Einsatz. Der Wasserstoff kann auch in Form eines Wasserstoffs enthaltenden Gases, d.h. mit Beimengungen anderer Inertgase, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Katalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

### Umsetzung in der Flüssigphase

Erfindungsgemäß erfolgt die Umsetzung von Ethylenglykol und/oder Monoethanolamin mit Ammoniak und einem Aminierungskatalysator in der Flüssigphase.

Im Rahmen der vorliegenden Erfindung bedeutet Umsetzung in der Flüssigphase, dass die Reaktionsbedingungen, wie Druck und Temperatur, so eingestellt werden, dass sowohl Ethylenglykol und Monoethanolamin in der Flüssigphase vorliegen und den Aminierungskatalysator flüssig umströmt.

Die Umsetzung von MEG und/oder MEA mit Ammoniak kann kontinuierlich oder diskontinuierlich durchgeführt werden. Bevorzugt ist eine kontinuierliche Umsetzung.

### Reaktoren

Geeignete Reaktoren für die Reaktion in der Flüssgiphase sind im Allgemeinen Rohrreaktoren. In den Rohrreaktoren kann der Katalysator als Fließ- oder Festbett angeordnet sein. Besonders bevorzugt erfolgt die Umsetzung von Ethylenglykol und/oder Monoethanolamin mit NH₃ in einem Rohrreaktor, in dem der Aminierungskatalysator als Festbett angeordnet ist.

Ist der Katalysator als Festbett angeordnet, kann es für die Selektivität der Reaktion vorteilhaft sein, den Katalysator im Reaktor mit inerten Füllkörpern zu vermischen, sie sozusagen zu "verdünnen". Der Anteil der Füllkörper in solchen Katalysatorzubereitungen kann 20 bis 80, bevorzugt 30 bis 60 und besonders bevorzugt 40 bis 50 Volumenteile betragen.

Alternativ erfolgt die Umsetzung vorteilhaft in einem Rohrbündelreaktor oder in einer Monostranganlage.Bei einer Monostranganlage kann der Rohrreaktor, in dem die Umsetzung erfolgt, aus einer Hintereinanderschaltung mehrerer (z.B. zweier oder dreier) einzelner Rohrreaktoren betehen. Optional ist hier vorteilhaft eine Zwischeneinspeisung von Feed (enthaltend das Edukt und/oder Ammoniak und/oder H₂) und/oder Kreisgas und/oder Reaktraustrag aus einem nachgeschalteten Reaktor möglich.

### Reaktionsbedingungen

Beim Arbeiten in der Flüssigphase leitet man bevorzugt das MEG und/oder MEA plus Ammoniak simultan in flüssiger Phase bei Drücken von im Allgemeinen 5 bis 30 MPa (50-300 bar), bevorzugt 5 bis 25 MPa, besonders bevorzugt 20 15 bis 25 MPa, und Temperaturen von im Allgemeinen 80 bis 350 °C, besonders 100 bis 300 °C, bevorzugt 120 bis 270 °C, besonders bevorzugt 130 bis 250 °C, insbesondere 160 bis 230 °C, inklusive Wasserstoff über den Katalysator, der sich üblicherweise in einem bevorzugt von außen beheizten Festbettreaktor befindet.

Der Wasserstoffpartialdruck beträgt vorzugsweise 0,25 bis 20 MPa (2,5 bis 200 bar), besonders bevorzugt 0,5 bis 15 MPa (5 bis 150 bar), ganz besonders bevorzugt 1 bis 10 MPa (10 bis 100 bar) und insbesondere bevorzugt 2 bis 5 MPa (20 bis 50 bar).

### Eintrag

ME und/oder MEA und Ammoniak werden dem Reaktor bevorzugt in flüssiger Form zugeführt und in flüssiger Form mit dem Aminierungskatalysator in Kontakt gebracht.

Es ist dabei sowohl eine Rieselfahrweise als auch eine Sumpffahrweise möglich.

Es ist zweckmäßig, die Reaktanden bereits vor der Zuführung in das Reaktionsgefäß zu erwärmen, und zwar bevorzugt auf die Reaktionstemperatur.

Ammoniak wird bevorzugt in der 0,90- bis 100-fachen molaren Menge, insbesondere in der 1,0-bis 20-fachen molaren Menge, jeweils bezogen auf das eingesetzte MEG und/oder MEA eingesetzt.

Die Katalysatorbelastung liegt im Allgemeinen im Bereich von 0,05 bis 5, bevorzugt 0, 1 bis 2, besonders bevorzugt 0,2 bis 0,6 kg (MEG + MEA) pro kg Katalysator und Stunde.

Die Bei den angegebenen Katalysatorbelastungen liegt der Umsatz von MEG bzw. MEA in der Regel im Bereich von 20 bis 75%, bevorzugt im Bereich von 30 bis 60% und ganz besonders bevorzugt im Bereich von 35 bis 55%.

Das im Zuge der Umsetzung gebildete Reaktionswasser jeweils ein Mol pro Mol umgesetzte Alkoholgruppe wirkt sich im Allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

### Austrag

Der Austrag aus dem Aminierungsreaktor enthält die Produkte der Aminierungsreaktion, nicht umgesetzte Edukte, wie Ethylenglykok und Ammoniak, sowie Wasserstoff und Wasser.

Als Produkte der Aminierungsreaktion enthält der Austrag aus dem Aminierungsreaktor weiterhin die entsprechenden Ethanolamine und/oder Ethylenamine auf Basis von MEG.

Bevorzugt enthält der Austrag aus dem Aminierungsreaktor MEA und/oder EDA.

Als Produkte der Aminierungsreaktion enthält der Reaktionsaustrag weiterhin bevorzugt höhere, lineare Ethylenamine der allgemeinen Formel

R-CH₂-CH₂-NH₂,

wobei R ein Rest der Formel -(NH-CH₂-CH₂)_{X}-NH₂ ist, wobei x eine ganze Zahl im Bereich von 1 bis 4, bevorzugt 1 bis 3 uns ganz besonders bevorzugt 1 bis 2 ist. Bevorzugt enthält der Reaktionsaustrag, DETA , TETA und TEPA, ganz besonders bevorzugt DETA und TETA und insbesondere bevorzugt DETA.

Als Produkte der Aminierungsreaktion kann der Austrag aus dem Aminierungsreaktor auch höhere, lineare Ethanolamine der Formel

R-CH₂-CH₂-OH

enthalten, wobei R ein Rest der Formel -(NH-CH₂-CH₂)_{X}-NH₂ ist, wobei x eine ganze Zahl im Bereich von 1 bis 4, bevorzugt 1 bis 3 uns ganz besonders bevorzugt 1 bis 2 ist. Ein Beispiel für ein höheres, lineares Ethanolamin ist AEEA.

Als Produkte der Aminierungsreaktion kann der Reaktionsaustrag kann auch zyklische Ethanolamine der Formel 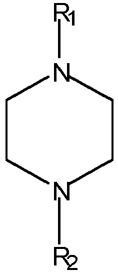 enthalten, wobei R₁ ein Rest der Formel -(CH₂-CH₂-NH)_{X}-CH₂-CH₂-OH ist, wobei x eine ganze Zahl im Bereich von 0 bis 4, bevorzugt 0 bis 3 und besonders bevorzugt 1 bis 2 ist, und R₂ unabhängig oder gleichzeitiger entweder H oder ein Rest der Formel -(CH₂-CH₂-NH)_{X}-CH₂-CH₂-OH ist, wobei x eine ganze Zahl im Bereich von 0 bis 4, bevorzugt 0 bis 3 und besonders bevorzugt 1 bis 2 ist oder ein Rest der Formel -(CH₂-CH₂-NH)_{X}-CH₂-CH₂-NH₂ ist, wobei x eine ganze Zahl im Bereich von 0 bis 4, bevorzugt 0 bis 3 und besonders bevorzugt 1 bis 2 ist. Ein Beispiel für ein zyklisches Ethanolamin ist Hydroxyethylpiperazin (HEP).

Als Produkte der Aminierungsreaktion kann der Reaktionsaustrag kann auch zyklische Ethylenamine der allgemeinen Formel 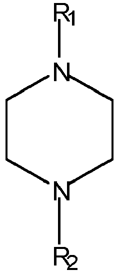 enthalten, wobei R₁ und R₂ unabhängig oder gleichzeitiger entweder H oder ein Rest der Formel -(CH₂-CH₂-NH)_{X}-CH₂-CH₂-NH₂ sein kann, wobei X eine ganze Zahl im Bereich von 0 bis 4, bevorzugt 0 bis 4 und besonders bevorzugt 1 bis 2 ist.

Beispiele für zyklische Ethylenamine, die im Reaktionsaustrag enthalten sind, sind Piperazin und AEPIP.

Bevorzugt enthält der Austrag 1 bis 60 Gew.-% MEA. 1 bis 90 Gew.-% EDA, 0,1 bis 30 Gew.-% höhere zyklische Ethylenamine, wie PIP und AEPIP, 0,1 bis 30 Gew.-% höhere lineare Ethylenamine, wie DETA, TETA und TEPA.

Besonders bevorzugt enthält der Austrag 10 bis 50 Gew.-% MEA. 25 bis 85 Gew.-% EDA, 0,25 bis 10 Gew.-% zyklische Ethylenamine, wie PIP und AEPIP, 1 bis 30 Gew.-% höhere lineare Ethylenamine, wie DETA, TETA und TEPA.

Ganz esonders bevorzugt enthält der Austrag 15 bis 45 Gew.-% MEA. 30 bis 70 Gew.-% EDA, 0,5 bis 5 Gew.-% zyklische Ethylenamine, wie PIP und AEPIP, 5 bis 25 Gew.-% höhere lineare Ethylenamine, wie DETA, TETA und TEPA.

Durch das erfindungsgemäße Verfahren können Selektivitätsquotienten SQ von 1,5 und mehr, bevorzugt 4 und mehr und besonders bevorzugt von 8 und mehr erreicht werden. Dies bedeutet, dass das Produktverhältnis von gewünschten linearen Ethylenaminen und Ethanolaminen, wie MEA und EDA zu nicht gewünschten zyklischen Ethylenaminen und nicht gewünschten höheren Ethanolaminen, wie PIP und AEEA, durch das erfindungsgemäße Verfahren erhöht werden kann.

Der Austrag wird in der Regel aufgearbeitet, so dass die verschiedenen Komponenten voneinander getrennt werden.

Dazu wird der Reaktionsaustrag zweckmäßiger Weise entspannt.

Die Komponenten, die nach der Entspannung in gasförmigen Form vorliegen, wie Wasserstoff und Inertgase, werden in der Regel in einem Gas-Flüssigabscheider von den flüssigen Komponenten getrennt. Die gasförmigen Komponenten können einzeln (nach einem weiteren Aufarbeitungsschritt) oder zusammen in den Aminierungsreaktor zurückgeführt werden.

Nach der Abtrennung von Wasserstoff und/oder Inertgas enthält der Austrag aus dem Aminerungsreaktor ggf. Ammoniak, nicht umgesetztes Ethylenglykol bzw. Monoethanolamin, Wasser sowie die Aminierungsprodukte.

Bevorzugt erfolgt die Auftrennung des Austrags aus dem Aminierungsreaktor in zwei Trennsequenzen, wobei jede Trennsequenz eine mehrstufige Destillation umfasst. Eine solche Aufarbeitung ist beispielsweise in der EP-B1-198699 beschrieben. Demgemäß werden in der ersten Trennsequenz werden zunächst Wasser und Ammoniak abgetrennt und in der zweiten Trennsequenz eine Auftrennung in nicht umgesetztes MEG, sowie MEA, EDA, PIP, DETA, AEEA und höhere Ethylenamine. Hierbei werden zunächst gegenüber dem Azeotrop von MEG und DETA niedriger sowie höher siedende Komponenten abgetrennt und anschließend das an MEG und DETA aufkonzentrierte Gemisch per Extraktivdestillation mit Triethylenglykol (TEG) als selektiven Lösungsmittel in einen MEG und DETA enthaltenden Strom aufgetrennt.

MEA kann teilweise oder vollständig, ggf. zusammen oder getrennt mit nicht umgesetztem MEG, in das erfindungsgemäße Verfahren zurückgeführt werden.

### Vorteile

In dem erfindungsgemäßen Verfahren ist es möglich MEG und/oder MEA mit einer hohen Selektivtät für die linearen Aminierungsprodukte DETA und EDA umzusetzen, während die Selektivität für das zyklische Aminierungsprodukt PIP und das höhere Ethanolamin AEEA gering ist. Ein Maß für diesen Effekt ist der Selektivitätsquotient SQ, der als Quotient aus der Summe der Selektivitäten von MEA und EDA und der Summe der Selektivitäten von PIP und AEEA (SQ=(S(DETA)+S(EDA))/(S(PIP)+S(AEEA)) definiert ist.

Die Erzielung eines hohen Selektivitätsquotienten SQ ist technisch vorteilhaft, da die Marktnachfrage nach den linearen Aminierungsprodukten MEA und EDA, sowie deren lineare Homologe, wie DETA und TETA, höher ist als die Nachfrage nach PIP bzw. AEEA.

Weiterhin werden durch das erfindungsgemäße Verfahren weniger unerwünschte Nebenprodukte gebildet. Unerwünschte Nebenprodukte sind beispielsweise gasförmige Zersetzungsprodukte oder unlösliche oder schwerlösliche Oligomere und Polymere auf Basis von MEA und EDA. Die Bildung solcher Nebenprodukte führt zu einer Verringerung der Kohlenstoffbilanz und zu damit zu einer Verringerung der Wirtschaftlichkeit des Verfahrens. Die Bildung von schwerlöslichen oder unlöslichen Nebenprodukte kann zu Ablagerung auf den Aminierungskatalysatoren führen, die die Aktivität der Aminierungskatalysatoren verringert.

Das erfindungsgemäße Verfahren führt ebenfalls zu einer Verringerung der Menge an N-Methylethylendiamin (NMEDA). NMEDA ist ein unerwünschtes Nebenprodukt. In vielen technischen Anwendungen wird eine Reinheit von EDA spezifiziert, bei der Anteil an NMEDA unter 500 Gew.-ppm liegt.

Weiterhin wurde festgestellt, dass die in das erfindungsgemäße Verfahren eingesetzten Katalysatorvorläufer in dem Verfahren eine hohe Aktivität aufweisen, so dass eine günstige Raum-Zeit-Ausbeute erzielt werden kann.

Insgesamt kann durch das erfindungsgemäße Verfahren ein vorteilhaftes Eigenschaftsspektrum in Bezug auf Gesamtselektivität, Selektivitätsquotient, Aktivität und die Bildung von unerwünschten Nebenprodukten erzielt werden.

Mit dem offenbarten Verfahren zur Herstellung von Aminierungskatalysatoren konnte ein Verfahren zur Verfügung gestellt werden, das hohen Sicherheitsstandards genügt.

Die Erfindung wird anhand der folgenden Beispiele erläutert:
Herstellung der Katalysatorvorläufer

Vergleichsbeispiel 1:
85,62 g Cobaltnitrathexahydrat wurde in ca. 80 ml heißem VE-Wasser gelöst und 269,75 g Ru-nitrosylnitratlösung (16 Gew.-% Ru) dazugeben. Die so erhaltene Lösung wurde mit VE-Wasser auf insgesamt 371 ml aufgefüllt.

Die so erhaltene Metallsalzlösung wurde in ein Sprühgefäß überführt.

500 g Al2O3-Träger (1-2 mm Splitt) wurden bei 900°C unter Luftatmosphäre calciniert. Im Anschluss wurde die maximale Wasseraufnahme des Trägers bestimmt. Diese betrug 0,78 ml/g. Der Splitt wurde der der zuvor hergestellten Metallsalzlösung getränkt. Die Menge der Lösung entspricht 95% der maximalen Wasseraufnahme des Splitts.

Der mit der Metallsalzlösung getränkte Splitt wurde anschließend 12 h bei 120°C im Umlufttrockenschrank getrocknet.

Im Anschluss an die Trocknung wurde der Katalysatorvorläufer bei 600°C in Gegenwart von Luft oxidativ calciniert.

Im Anschluss an die oxidative Kalzinierung wurde der Katalysator reduziert, in dem bei 200°C der Katalysatorvorläufer mit einem Gasstrom aus Wasserstoff für ca. 6 Stunden umströmt wurde.

Im Anschluss an die Reduktion wurde der Katalysator passiviert, in dem bei Raumtemperatur der Katalysator mit einem Gasstrom von 98 NL/h N2 und 2 NL/h Luft umströmt wurde. Die Luftmenge wurde langsam erhöht, während die N2-Menge langsam verringert wurde, bis 20 NL/h N2 und 18 NL/h Luft erreicht wurden. Die Luftmengenerhöhung wurde so durchgeführt, dass die Katalysatortemperatur 35°C nicht überstieg.

Vergleichsbeispiel 2:
Der Katalysatorvorläufer wurde gemäß Beispiel B3 der WO 2013/072289 hergestellt. Demgemäß wurden die Katalysatorvorläufer bei einer Temperatur von 450°C unter Durchleiten von Luft oxidativ calciniert- Vor der Reduktion der so hergestellten Tabletten, wurden diese zu 1-2 mm Splitt zerkleinert.

Der so erhaltene Katalysatorvorläufer wurde gemäß der folgenden Vorschrift reduziert (siehe Tabelle 1):

**Tabelle 1:**

| | Dauer (min) | Temperatur (°C) | Stickstoff (Nl/h) | Wasserstoff (Nl/h) | Luft (Nl/h) | Bemerkung |
|---|---|---|---|---|---|---|
| 1 | 30min | RT | 100 | - | - | Spülvorgang bei RT |
| 2 | 44min | 220 | 95 | 5 | - | Aufheizen auf 220°C |
| 3 | 120min | 220 | 95 | 5 | - | Haltezeit bei 220°C |
| 4 | 30min | 280 | 95 | 5 | - | Aufheizen auf 280°C |
| 5 | 15min | 280 | 95 | 5 | - | Erhöhung der Wasserstoffmenge |
| 6 | 15min | 280 | 90 | 10 | - | Erhöhung der Wasserstoffmenge |
| 7 | 15min | 280 | 80 | 20 | | Erhöhung der Wasserstoffmenge |
| 8 | 15min | 280 | 70 | 30 | | Erhöhung der Wasserstoffmenge |
| 9 | 15min | 280 | 60 | 40 | | Erhöhung der Wasserstoffmenge |
| 10 | 15min | 280 | 50 | 50 | | Abkühlvorgang auf RT |
| 11 | 120min | 280 | 50 | 50 | | Haltezeit bei 280°C |

Im Anschluss an die Reduktion erfolgte eine Passivierung des Katalysatorvorläufers. Hierzu wurde der reduzierte Katalysatorvorläufer mit einem Strom aus 50 NL/h N2 und 0 NL/h Luft umströmt. Die Luftmenge wurde langsam erhöht, während die N2-Menge langsam verringert wurde, bis 20 NL/h N2 und 20 NL/h Luft erreicht sind. Die Luftmengenerhöhung wurde so durchgeführt, dass die Katalysatortemperatur 35 °C nicht überstieg.

Vergleichsbeispiel 3:
Der Katalysatorvorläufer wurde gemäß Beispiel B3 der WO 2013/072289 hergestellt.

Die so erhaltenen Tabletten (3*3 mm) wurden zu 1-2 mm Splitt zerkleinert. Die Wasseraufnahme des Splitts betrug 0,25mL/g.

Es wurde eine Metallsalzlösung hergestellt. Hierzu wurden 9,39 g Cobaltnitrathexahydrat (20,25 Gew.-% Co) in heißem Wasser gelöst und 24,58 g Ru-nitrosylnitratlösung zugegeben. Die so erhaltene Lösung wurde mit VE-Wasser auf 45 ml aufgefüllt und in ein Sprühgefäß überführt.

Der Splitt wurde in einer Tränkapparatur mit einer Menge besprüht, die 90% der maximalen Wasseraufnahme des Splitts entspricht. Im Anschluss wurde der Katalysatorsplitt für 16 h bei 120°C im Umlufttrockenschrank getrocknet.

Im Anschluss an die Trocknung wurde der Katalysatorvorläufer bei 600°C in Gegenwart von Luft oxidativ calciniert.

Der so erhaltene Katalysatorvorläufer wurde gemäß der folgenden Vorschrift reduziert (siehe Tabelle 2):

**Tabelle 2:**

| | Dauer (min) | Temperatur (°C) | Stickstoff (Nl/h) | Wasserstoff (Nl/h) | Luft (Nl/h) | Bemerkung |
|---|---|---|---|---|---|---|
| 1 | 30min | RT | 100 | - | - | Spülvorgang bei RT |
| 2 | 44min | 220 | 95 | 5 | - | Aufheizen auf 220°C |
| 3 | 120min | 220 | 95 | 5 | - | Haltezeit bei 220°C |
| 4 | 30min | 280 | 95 | 5 | - | Aufheizen auf 280°C |
| 5 | 15min | 280 | 95 | 5 | - | Erhöhung der Wasserstoffmenge |
| 6 | 15min | 280 | 90 | 10 | - | Erhöhung der Wasserstoffmenge |
| 7 | 15min | 280 | 80 | 20 | | Erhöhung der Wasserstoffmenge |
| 8 | 15min | 280 | 70 | 30 | | Erhöhung der Wasserstoffmenge |
| 9 | 15min | 280 | 60 | 40 | | Erhöhung der Wasserstoffmenge |
| 10 | 15min | 280 | 50 | 50 | | Abkühlvorgang auf RT |
| 11 | 120min | 280 | 50 | 50 | | Haltezeit bei 280°C |

Im Anschluss an die Reduktion erfolgte eine Passivierung des Katalysatorvorläufers. Hierzu wurde der reduzierte Katalysatorvorläufer mit einem Strom aus 50 NL/h N2 und 0 NL/h Luft umströmt. Die Luftmenge wurde langsam erhöht, während die N2-Menge langsam verringert wurde, bis 20 NL/h N2 und 20 NL/h Luft erreicht sind. Die Luftmengenerhöhung wurde so durchgeführt, dass die Katalysatortemperatur 35 °C nicht überstieg.

Beispiel 1:

85,62 g Cobaltnitrathexahydrat wurde in ca. 80 ml heißem VE-Wasser gelöst und 269,75 g Ru-nitrosylnitratlösung (16 Gew.-% Ru) dazugeben. Die so erhaltene Lösung wurde mit VE-Wasser auf insgesamt 371 ml aufgefüllt.

Die so erhaltene Metallsalzlösung wurde in ein Sprühgefäß überführt.

500 g Al2O3-Träger (1-2 mm Splitt) wurden bei 900°C unter Luftatmosphäre calciniert. Im Anschluss wurde die maximale Wasseraufnahme des Trägers bestimmt. Diese betrug 0,78 ml/g. Der Splitt wurde der der zuvor hergestellten Metallsalzlösung getränkt. Die Menge der Lösung entspricht 95% der maximalen Wasseraufnahme des Splitts.

Der mit der Metallsalzlösung getränkte Splitt wurde anschließend 12 h bei 120°C im Umlufttrockenschrank getrocknet.

Im Anschluss an die Trocknung wurde der Katalysatorvorläufer unter den in Tabelle 1 aufgeführten Bedingungen reduktiv calciniert.

**Tabelle 3:**

| | Dauer (min) | Temperature (°C) | Aufheizrate (°C/min) | Gas Flow I Gasfluss (NL/h) | | | Bemerkung |
|---|---|---|---|---|---|---|---|
| | | | | Stickstoff | Wasserstoff | Luft | |
| 1 | 30min | RT | Ohne | 100 | - | - | Spülvorgang bei RT |
| 2 | 150min | 150 | 1 | 95 | 5 | - | Aufheizen auf 150°C |
| 3 | 120min | 150 | Ohne | 95 | 5 | - | Haltezeit bei 150°C |
| 4 | 50min | | 1 | 95 | 5 | - | Aufheizen auf 150°C |
| 5 | 15min | 200 | Ohne | 95 | 5 | - | Erhöhung der Wasserstoffmenge |
| 6 | 15min | 200 | Ohne | 90 | 10 | - | Erhöhung der Wasserstoffmenge |
| 7 | 15min | 200 | Ohne | 80 | 20 | | Erhöhung der Wasserstoffmenge |
| 8 | 15min | 200 | Ohne | 70 | 30 | | Erhöhung der Wasserstoffmenge |
| 9 | 15min | 200 | Ohne | 60 | 40 | | Erhöhung der Wasserstoffmenge |
| 10 | 15min | 200 | Ohne | 50 | 50 | | Abkühlvorgang auf RT |
| 11 | 120min | 200 | Ohne | 50 | 50 | | Haltezeit bei 200°C |

Im Anschluss an die reduktive Kalzinierung wurde der Katalysator passiviert, in dem bei Raumtemperatur der Katalysator mit einem Gasstrom von 98 NL/h N2 und 2 NL/h Luft umströmt wurde. Die Luftmenge wurde langsam erhöht, während die N2-Menge langsam verringert wurde, bis 20 NL/h N2 und 18 NL/h Luft erreicht wurden. Die Luftmengenerhöhung wurde so durchgeführt, dass die Katalysatortemperatur 35°C nicht überstieg.

### Beispiel 2:

Ein Katalysatorvorläufer wurde gemäß Beispiel B3 der WO 2013/072289 hergestellt.

Die so erhaltenen Tabletten (3*3 mm) wurden zu 1-2 mm Splitt zerkleinert. Die Wasseraufnahme des Splitts betrug 0,30mL/g.

Es wurde eine Metallsalzlösung hergestellt. Hierzu wurden 20,25 g Cobaltnitrathexahydrat (20,25 Gew.-% Co) in heißem Wasser gelöst und 37,91 g Ru-nitrosylnitratlösung zugegeben. Die so erhaltene Lösung wurde mit VE-Wasser auf 71 ml aufgefüllt und in ein Sprühgefäß überführt.

Der Splitt wurde in einer Tränkapparatur mit einer Menge besprüht, die 95% der maximalen Wasseraufnahme des Splitts entspricht. Um die homogene Aufnahme der Tränklösung zu gewährleisten, wurde der Splitt noch weitere 30 min nachrotiert.

Im Anschluss wurde der Katalysatorsplitt für 16 h bei 120°C im Umlufttrockenschrank getrocknet.

Der so erhaltene Katalysatorvorläufer wurde wie in Beispiel 1 beschrieben reduktiv calciniert und passiviert.

Katalysatortestung:
Die Testung der Katalysatoren erfolgte in einer kontinuierlich betriebenen Mehrfachanlage im Technikumsmaßstab. Der Reaktionsteil der Anlage besteht aus acht Einzelreaktoren, von denen jeweils vier in einem Reaktorblock (Heizblock) zusammengefasst sind. Jeder Einzelreaktor ist ein 1,5 m langes Edelstahlrohr mit einem Innendurchmesser von 8 mm. Die Rohre sind in einem elektrisch beheizten Reaktorblock bestehend aus einer Al-Mg-Legierung eingebaut.

Der Katalysator wurde in Form von Splitt (1,5 mm - 2 mm) in den Reaktor eingefüllt und auf einer ca. 33 cm langen Inertschüttung bestehend aus 3 mm großen Glasperlen gelagert. Oberhalb des Katalysatorbetts schließt sich eine weitere, 15 cm lange Inertschüttung bestehend aus 3 mm großen Glasperlen an.

Der Katalysator und die Inertschüttung wurden im Reaktor durch einen 1 cm langen Gewebedraht fixiert.

Jeder Reaktor wurde im geraden Durchgang betrieben und von unten angeströmt.

Das flüssige Edukt wurde aus einer Vorlage mit Hilfe einer HPLC-Pumpe zugeführt. Die Zuführung von Wasserstoff, Stickstoff und Ammoniak erfolgte durch separate Rohrleitungen.

Proben der flüssigen Reaktorausträge wurden hinter dem Reaktorausgang aus einem Abscheider entnommen. Die Reaktorausträge wurden gaschromatographisch analysiert.

Die Aktivierung des Katalysators erfolgte vor der Reaktion bei 200 °C und 170 bar über eine Zeit von 18 h in einem 50:50-Gemisch aus Wasserstoff und Stickstoff.

Die Testung aller Katalysatoren erfolgte unter folgenden Bedingungen:
- Temperatur: 165 °C
- Druck: 170 bar
- H2: 5 NL/h
- N2: 10 NL/h
- Molares Verhältnis NH3 : MEG = 10 : 1
- Katalysatorbelastung: 0,3 kg/L/h - 0,5 kg/L/h
- Katalysatorvolumen: 50 mL

Die genauen Bedingungen sind in der nachfolgenden Tabelle 4 zusammengefasst.

**Tabelle 4:**

| Katalysator | Kat.belastung /kg/L/h | Umsatz / FI.-% | EDA / FI.-% | DETA / FI.-% | AEEA / FI.-% | PIP / FI.-% | MEA / FI.-% | NMEDA + NEEDA + EA / FI.-% | Ges.Sel. (5 Hauptprodukte) / FI.-% | (EDA+DETA) /(PIP+AEEA) |
|---|---|---|---|---|---|---|---|---|---|---|
| Vergleichs beispiel 1 | 0,3 | 15,2 | 7,2 | 0,2 | 0,2 | 0,4 | 6,9 | 0,0 | 98,1 | 11,7 |
| Vergleichs beispiel 2 | 0,3 | 27,0 | 11,6 | 0,9 | 0,9 | 1,6 | 11,4 | 0,0 | 97,9 | 5,0 |
| Vergleichs beispiel 3 | 0,3 | 30,3 | 13,5 | 1,2 | 1,1 | 2,6 | 11,1 | 0,0 | 97,4 | 4,0 |
| Beispiel 1 | 0,3 | 46,6 | 20,1 | 2,3 | 2,2 | 5,7 | 11,7 | 2,5 | 90,2 | 2,9 |
| Beispiel 2 | 0,3 | 41,4 | 15,9 | 3,2 | 2,1 | 7,4 | 10,5 | 0,2 | 94,5 | 2,0 |

In Vergleichsbeispiel 1 wurde ein Katalysatorenvorläufer (ein mit Ru und Co getränkter Alumniumoxid-Trägermaterial)oxidativ calciniert.

Beispiel 1 unterscheidet sich von Vergleichsbeispiel daduch, dass der Katalysatorvorläufer nicht oxidativ, sondern reduktiv calciniert wurde.

Es zeigt sich, dass durch die reduktive Calcinierung der Umsatz drastisch erhöht werden konnte. Der Selektivitätsquotient liegt trotz des hohen Umsatzes bei 2,9, so dass bei der erfindungsgemäßen Umsetzung im hohen Maße die gewünschten Produkte EDA und DETA erhalten werden und im geringeren Maße die unerwünschten Produkte PIP und AEEA erhalten werden. Gleichzeitig werden nur geringe Mengen an unerwünschten Nebenprodukten, wie NMEDA, gebildet.

In Vergleichsbeispiel 2 wurde ein der Katalysatorvorläufer enthaltend Ni, Co, Cu und Sn auf Aluminiumoxid nach der Trockung in Gegenwart von Luft bei 450°C oxidativ calciniert.

In Vergleichsbeispiel 3 wurde ein der oxidativ calcinierte Katalysatorvorläufer aus Vergleichsbeispiel 2 mit Ru und Co nachgetränkt und der so erhaltene getränkte Katalysatorvorläufer oxidativ calciniert.

Beispiel 2 unterscheidet sich von Beispiel 3 dadurch, dass der mit Ru und Co getränkte Katalysatorvorläufer reduktiv calciniert wurde.

Es zeigt sich, dass durch die reduktive Calcinierung der Umsatz drastisch erhöht werden konnte. Der Selektivitätsquotient liegt trotz des hohen Umsatzes bei 2,0, so dass bei der erfindungsgemäßen Umsetzung im hohen Maße die gewünschten Produkte EDA und DETA erhalten werden und im geringeren Maße die unerwünschten Produkte PIP und AEEA erhalten werden. Gleichzeitig werden nur geringe Mengen an unerwünschten Nebenprodukten, wie NMEDA, gebildet.

Ein Vergleich von Beispiel 1 und Beispiel 2 zeigt, dass bei Katalysatorvorläufer, die durch Tränkung eines katalytischen Trägermaterials (Beispiel 2) anstelle eines konventionellenTrägermaterials (Beispiel 1) hergestellt und anschließend reduktiv calciniert werden, die Selektivität nochmals erhöht werden kann. Gleichzeitig kann die Menge unerwünschten Nebenprodukten, wie NMEDA, gesenkt werden.

## Patentansprüche

1. Verfahren zur Herstellung von Alkanolaminen und/oder Ethylenaminen in der Flüssigphase, durch Umsetzung von Ethylenglykol und/oder Monoethanolamin mit Ammoniak in Gegenwart eines Aminierungskatalysators, der ein oder mehrere Aktivmetalle ausgewählt aus Sn und den Elementen der Gruppen 8, 9, 10 und 11 des Periodensystems der Elemente enthält, **dadurch gekennzeichnet, dass** die Herstellung des Aminierungskatalysators folgende Schritte umfasst:
(i) Herstellung eines Katalysatorvorläufers durch Inkontaktbringen eines konventionellen oder katalytischen Trägermaterials mit ein oder mehreren löslichen Verbindungen der Aktivmetalle und optional ein oder mehreren löslichen Verbindungen von Katalysatorzusatzelementen;
(ii) reduktive Calcinierung des in Schritt (i) erhaltenen Katalysatorvorläufers, wobei die reduktive Calcinierung in Gegenwart von Wasserstoff erfolgt und der Sauerstoffgehalt bei der reduktiven Calcinierung weniger als 0,1 Vol.-% beträgt;.
(iii) Passivierung des reduktiv calcinierten Katalysatorvorläufers aus Schritt (ii);
(iv) Aktivierung des passivierten Katalysators aus Schritt (iii).

2. Verfahren nach Anspruch 1, **dadurch gekennzeichnet, dass** das Inkontaktbringen des katalytischen oder konventionellen Trägermaterials mit den löslichen Verbindungen der Aktivmetalle und ggf. mit den löslichen Verbindungen der Katalysatorzusatzelemente durch Tränkung oder Imprägnierung erfolgt.

3. Verfahren nach mindesten einem der Ansprüche 1 bis 2, **dadurch gekennzeichnet, dass** die ein oder mehreren Aktivmetalle ausgewählt werden aus der Gruppe bestehend aus Co, Ru, Sn, Ni und Cu.

4. Verfahren nach mindestens einem der Ansprüche 1 bis 3, **dadurch gekennzeichnet, dass** ein der ein oder mehreren Aktivmetalle Ru oder Co ist.

5. Verfahren nach mindestens einem der Ansprüche 1 bis 4, **dadurch gekennzeichnet, dass** die löslichen Verbindungen der Aktivmetalle, die mit den Trägermaterial in Kontakt gebracht werden, teilweise oder vollständig in Form ihrer Nitrate oder Nitrosylnitrate eingesetzt werden.

6. Verfahren nach mindestens einem der Ansprüche 1 bis 5, **dadurch gekennzeichnet, dass** das Trägermaterial Al und/oder Zr enthält.

7. Verfahren nach mindestens einem der Ansprüche 1 bis 6, **dadurch gekennzeichnet, dass** das Trägermaterial gleichzeitig oder nacheinander mit einer löslichen Ru-Verbindung und einer löslichen Co-Verbindung in Kontakt gebracht werden, wobei die lösliche Cobaltverbindung Co-Nitrat ist.

8. Verfahren nach mindestens einem der Ansprüche 1 bis 7, **dadurch gekennzeichnet, dass** der Reaktor in dem die reduktive Calcinierung durchgeführt wird, ein Schachtreaktor, Drehrohrofen, Etagenofen oder Wirbelschichtreaktor ist.

9. Verfahren nach mindestens einem der Ansprüche 1 bis 8, **dadurch gekennzeichnet, dass** die Temperatur bei der reduktiven Calcinierung im Bereich von 100 bis 300°C liegt.

10. Verfahren nach mindestens einem der Ansprüche 1 bis 9, **dadurch gekennzeichnet, dass** die reduktive Calcinierung in einem Reaktor erfolgt, der mit einer Denox-Anlage verbunden ist.

11. Verfahren nach mindestens einem der Ansprüche 1 bis 10, **dadurch gekennzeichnet, dass** die Temperatur bei der Aktivierung im Bereich von 100 bis 300°C liegt.

## Claims

1. A process for preparing alkanolamines and/or ethyleneamines in the liquid phase, by reacting ethylene glycol and/or monoethanolamine with ammonia in the presence of an amination catalyst comprising one or more active metals selected from Sn and the elements of groups 8, 9, 10 and 11 of the Periodic Table of the Elements, wherein the preparation of the amination catalyst comprises the following steps:
(i) preparing a catalyst precursor by contacting a conventional or catalytic support material with one or more soluble compounds of the active metals and optionally one or more soluble compounds of added catalyst elements;
(ii) reductively calcining the catalyst precursor obtained in step (i), where the reductive calcination is effected in the presence of hydrogen and the oxygen content in the reductive calcination is less than 0.1% by volume;
(iii) passivating the reductively calcined catalyst precursor from step (ii);
(iv) activating the passivated catalyst from step (iii) .

2. The process according to claim 1, wherein the catalytic or conventional support material is contacted with the soluble compounds of the active metals and optionally with the soluble compounds of the added catalyst elements by soaking or impregnation.

3. The process according to at least one of claims 1 and 2, wherein the one or more active metals are selected from the group consisting of Co, Ru, Sn, Ni and Cu.

4. The process according to at least one of claims 1 to 3, wherein one of the one or more active metals is Ru or Co.

5. The process according to at least one of claims 1 to 4, wherein the soluble compounds of the active metals that are contacted with the support material are used partly or completely in the form of their nitrates or nitrosylnitrates.

6. The process according to at least one of claims 1 to 5, wherein the support material comprises Al and/or Zr.

7. The process according to at least one of claims 1 to 6, wherein the support material is contacted simultaneously or successively with a soluble Ru compound and a soluble Co compound, where the soluble cobalt compound is Co nitrate.

8. The process according to at least one of claims 1 to 7, wherein the reactor in which the reductive calcination is conducted is a shaft reactor, rotary furnace, staged furnace or fluidized bed reactor.

9. The process according to at least one of claims 1 to 8, wherein the temperature in the reductive calcination is in the range from 100 to 300°C.

10. The process according to at least one of claims 1 to 9, wherein the reductive calcination is effected in a reactor connected to a denox plant.

11. The process according to at least one of claims 1 to 10, wherein the temperature in the activation is in the range from 100 to 300°C.

## Revendications

1. Procédé pour la préparation d'alcanolamines et/ou d'éthylèneamines en phase liquide, par transformation d'éthylène glycol et/ou de monoéthanolamine avec de l'ammoniac en présence d'un catalyseur d'amination, qui contient un ou plusieurs métaux actifs choisis parmi Sn et les éléments des groupes 8, 9, 10 et 11 du système périodique des éléments, **caractérisé en ce que** la préparation du catalyseur d'amination comprend les étapes suivantes :
(i) préparation d'un précurseur de catalyseur par mise en contact d'un matériau de support conventionnel catalytique avec un ou plusieurs composés solubles des métaux actifs et éventuellement un ou plusieurs composés solubles d'éléments supplémentaires de catalyseur ;
(ii) calcination réductrice du précurseur de catalyseur obtenu dans l'étape (i), la calcination réductrice étant réalisée en présence d'hydrogène et la teneur en oxygène lors de la calcination réductrice étant inférieure à 0,1 % en volume ;
(iii) passivation du précurseur de catalyseur calciné de manière réductrice de l'étape (ii) ;
(iv) activation du catalyseur passivé de l'étape (iii) .

2. Procédé selon la revendication 1, **caractérisé en ce que** la mise en contact du matériau de support catalytique ou conventionnel avec les composés solubles des métaux actifs et éventuellement avec les composés solubles des éléments supplémentaires de catalyseur est réalisée par infusion ou imprégnation.

3. Procédé selon au moins l'une des revendications 1 et 2, **caractérisé en ce que** le ou les métaux actifs sont choisis dans le groupe constitué par Co, Ru, Sn, Ni et Cu.

4. Procédé selon au moins l'une des revendications 1 à 3, **caractérisé en ce qu'**un ou plusieurs métaux actifs est Ru ou Co.

5. Procédé selon au moins l'une des revendications 1 à 4, **caractérisé en ce que** les composés solubles des métaux actifs qui sont mis en contact avec le matériau de support sont utilisés partiellement ou totalement sous la forme de leurs nitrates ou nitrosylnitrates.

6. Procédé selon au moins l'une des revendications 1 à 5, **caractérisé en ce que** le matériau de support contient Al et/ou Zr.

7. Procédé selon au moins l'une des revendications 1 à 6, **caractérisé en ce que** le matériau de support est mis en contact simultanément ou consécutivement avec un composé soluble de Ru et un composé soluble de Co, le composé soluble de cobalt étant un nitrate de Co.

8. Procédé selon au moins l'une des revendications 1 à 7, **caractérisé en ce que** le réacteur dans lequel la calcination réductrice est mise en œuvre est un réacteur en forme de cage, un four tubulaire rotatif, un four à étages ou un réacteur à lit fluidisé.

9. Procédé selon au moins l'une des revendications 1 à 8, **caractérisé en ce que** la température lors de la calcination réductrice se situe dans la plage de 100 à 300 °C.

10. Procédé selon au moins l'une des revendications 1 à 9, **caractérisé en ce que** la calcination réductrice est réalisée dans un réacteur qui est relié avec une installation Denox.

11. Procédé selon au moins l'une des revendications 1 à 10, **caractérisé en ce que** la température lors de l'activation se situe dans la plage de 100 à 300 °C.
